# EUROPEAN PATENT APPLICATION

(11) **EP 4 821 930 A1**
(43) Date of publication of application: **14.10.2026**
(21) Application number: 24899822.1
(22) Date of filing: 03.12.2024
(51) Int. Cl.: C07D 471/20, C07D 471/04, C07D 487/04, A61K 31/437, A61K 31/519, A61P 35/00, A61P 35/02

(54) **SUBSTITUTED POLYCYCLIC COMPOUND**

(30) Priority: 04.12.2023 CN 202311648598; 18.04.2024 CN 202410472871; 04.07.2024 CN 202410895093; 03.09.2024 CN 202411230078
(71) Applicant: Shouyao Holdings (Beijing) Co., Ltd., Beijing 100176 (CN)
(72) Inventor: CHEN, Kuncheng, Beijing 100195 (CN); CHEN, Xi, Beijing 100195 (CN); HAO, Tiantian, Beijing 100195 (CN); LI, Xiaopeng, Beijing 100195 (CN); XU, Xinhe, Beijing 100195 (CN); WANG, Biting, Beijing 100195 (CN)
(74) Representative: f & e patent
(86) International application number: PCT/CN2024/136533
(87) International publication number: WO 2025/119184

(57) **Abstract**

The present application relates to a class of substituted polycyclic compounds having biological inhibitory activity as represented by formula (II), and preparation methods and uses thereof. The uses include the use of the compound of formula (II) in the preparation of a medicament for the treatment of MLL-related diseases. In the preparation process, the compound of the present invention is obtained through a series of reactions including substitution, cyclization, reduction, and deprotection.

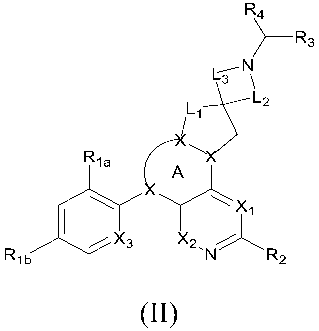

## Description

### CROSS-REFERENCE

This application claims priority to Chinese Patent Application No. 202311648598.0, filed on December 4, 2023, entitled "Substituted Polycyclic Compounds", Chinese Patent Application No. 202410472871.7, filed on April 18, 2024, entitled "Substituted Polycyclic Compounds", Chinese Patent Application No. 202410895093.2, filed on July 4, 2024, entitled "Substituted Polycyclic Compounds", and Chinese Patent Application No. 202411230078.2, filed on September 3, 2024, entitled "Substituted Polycyclic Compounds", the entire disclosures of which are incorporated herein by reference in their entireties.

### TECHNICAL FIELD

The present invention generally relates to novel substituted heterocyclic compounds having Menin-MLL interaction inhibitory activity, methods for their preparation, pharmaceutical compositions thereof, and also to the use of such compounds and pharmaceutical compositions thereof for the treatment of diseases that benefit from inhibiting the Menin-MLL interaction, for example, for the treatment of acute leukemia.

### BACKGROUND

The development of human acute leukemia is associated with genetic and epigenetic alterations (Figueroa 2010; Rodriguez 2011). Approximately 5% to 10% of pediatric and adult patients with acute leukemia harbor chromosomal translocations in the 11q23 region, leading to rearrangements of *KMT2A* (*MLL1*) (Bill 2020). The aggressive leukemia driven by MLL1 rearrangements (MLL-r) has a 5-year survival rate of only 35% (Dimartino 1999; Marschalek 2011). The incidence of such translocations is as high as 80% in infant acute leukemia patients (Brown 2013). MLL-r leukemia generally has a poor prognosis, particularly in infant acute lymphoblastic leukemia, and there remains a lack of more effective and less toxic treatments to date.

MLL1 is a histone lysine N-methyltransferase responsible for H3K4 methylation and is essential for the expression of the HOX family of genes, which are regulators of hematopoietic cell differentiation. MLL1 rearrangements generate MLL1 fusion proteins, leading to transcriptional dysregulation that drives the development of acute leukemia (Meyer 2018). MLL1 fusion proteins regulate target gene expression through interactions with chromatin-associated protein complexes. Menin protein is a critical MLL partner protein in this complex and plays an important role in the process by which MLL fusion proteins drive target gene expression, regulating the expression of MEIS1 and HOX family proteins (Chen 2006). The Menin protein has a binding pocket on its surface for MLL1, which can bind with high affinity to the conserved N-terminal sequence of MLL1. This interaction is essential for MLL1 fusion proteins to regulate transcription of target genes (Yokoyama 2005; Caslini 2007). Therefore, modulating the Menin-MLL interaction represents an important target for small-molecule therapy of MLL-r leukemia. Blocking the interaction between Menin and MLL1 fusion proteins using small molecule inhibitors has been demonstrated as a potential therapeutic strategy for the treatment of MLL-r leukemia.

To date, no Menin-MLL inhibitor has been approved for marketing. Abroad, six inhibitors have recently entered clinical studies one after another. Syndax's inhibitor SNDX-5613 (revumenib) first entered Phase I/II clinical studies (NCT04065399) on August 22, 2019, for the treatment of acute myeloid leukemia (AML), acute lymphoblastic leukemia (ALL), mixed lineage acute leukemia (MLAL), mixed phenotype acute leukemia (MPAL), and acute leukemia of ambiguous lineage (ALAL) harboring MLL rearrangements or NPM1 mutations. Immediately afterward, Kura's inhibitor KO-539 initiated Phase I/II clinical studies on August 26, 2019 (NCT04067336); Janssen initiated a Phase I clinical study of JNJ-75276617 on March 21, 2021 (NCT04065399); Daiichi Sankyo's inhibitor DS-1594 initiated a Phase I clinical study on March 25, 2021; Biomea's inhibitor BMF-219 initiated a Phase I clinical study on January 24, 2022; and Sumitomo's inhibitor DSP-5336 initiated a Phase I clinical study on February 28, 2022. Domestically, Yahu Pharmaceutical's BN104 initiated a Phase I clinical study on September 4, 2023. Bayer, Agios, the University of Michigan, the University of Pennsylvania, and others have also made patent filings.

Recently, based on the results of the AUGMENT-101 (NCT04065399) study, it was found that patients develop acquired resistance after treatment with the Menin inhibitor revumenib (Perner 2023). Next-generation sequencing of bone marrow samples from resistant patients revealed somatic mutations in Menin. The mutation sites included M327V or M327I, T349M, G331R, and S160T, with mutation frequencies ranging from 5.9% to 28.2%. PDX models showed that continuous treatment of mice with the Menin inhibitor VTP-50469 led to Menin mutations in 68% of relapsed samples, with mutation sites including MEN1^{M327V}, MEN1^{M327I}, MEN1^{G331R}, MEN1^{G331D}, MEN1^{T349M}, and MEN1^{S160C}, which are almost identical to the mutation sites observed in patients. Crystal structures revealed that the three high-frequency mutation residues, M327, G331, and T349, are located near residue W346, affecting the formation of a hydrogen bond between revumenib and the indole N-H of W346, thereby reducing the binding affinity of revumenib to the Menin protein. However, these mutations do not affect the binding interface between Menin and MLL. Cellular and *in vitro* binding assays similarly demonstrated that the binding affinity of Menin mutant proteins to revumenib is significantly reduced, thereby preventing revumenib-mediated regulation of leukemia gene expression by the Menin-MLL1 protein complex.

Therefore, developing next-generation Menin inhibitors targeting drug-resistant Menin mutations will effectively overcome the problem of drug resistance and relapse in patients, representing a significant clinical need. Using computer-aided drug design, we discovered structurally novel compounds and, through structural modification, obtained new compounds with potent activity against Menin drug-resistant mutations and favorable pharmaceutical properties.

### SUMMARY OF THE INVENTION

The present invention provides a compound represented by formula (II), or a pharmaceutically acceptable salt, solvate, polymorph, tautomer, metabolite, or prodrug thereof, 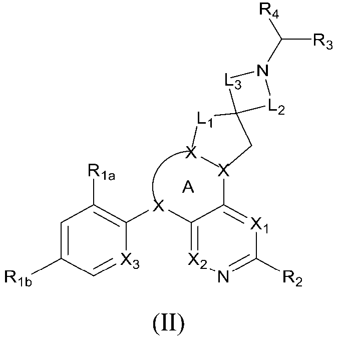 wherein,
Ring A is a 5- to 8-membered nitrogen-containing heterocyclic ring or nitrogen-containing heteroaromatic ring, each X is independently C or N,
R₁ₐ is H, -C(=O)-NR₂ₐR_{2b}, 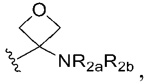 phenyl, or 5-6-membered heteroaryl, the heteroaryl contains one oxygen atom and/or one sulfur atom and/or 1-3 nitrogen atoms, and optionally contains carbonyl, and the phenyl and heteroaryl are optionally substituted by 3-6-membered cycloalkyl or C₁₋₄ alkyl,
R₂ₐ and R_{2b} are each independently H or C₁₋₄ alkyl, the alkyl is optionally substituted with -OH or -NH₂,
R_{1b} is F or Cl,
R₂ is selected from H, halogen, C₁₋₄ alkyl, -O-C₁₋₄ alkyl and -NR₃ₐR_{3b},
R₃ₐ and R_{3b} are each independently H or C₁₋₄ alkyl,
X₁ and X₂ are each independently N or CH,
X₃ is N or R₁₀,
R₁₀ is H, halogen, C₁₋₆ alkyl, or -O-C₁₋₆ alkyl,
L₁ is -CH₂-, -CH₂-CH₂- or -CH₂-CH₂-CH₂-,
L₂ and L₃ are each independently -CH₂- or -CH₂-CH₂-,
R₄ is C₁₋₆ alkyl, 3-8-membered cycloalkyl or 3-8-membered heterocycloalkyl,
R₃ is -C₁₋₆ alkylene -NR₄ₐR_{4b}, -C₁₋₆ alkylene -C(O)-NR₅ₐR_{5b}, -C₁₋₆ alkylene -OH, or -C₁₋₆ alkylene -NR₇-C(O)-O-C₁₋₄ alkylene -O-C(O)-C₁₋₄ alkyl, wherein the alkyl or alkylene are optionally substituted with halogen, -CN, -OH or -O-C₁₋₄ alkyl,
R₄ₐ and R_{4b} are each independently selected from H, C₁₋₆ alkyl, -C(O)-C₁₋₄ alkyl, -C(O)-O-C₁₋₄ alkyl and -C(O)-NR₈ₐR_{8b}, the C₁₋₆ alkyl is optionally substituted with halogen, -CN, -OH, -S(O)₂-C₁₋₄ alkyl, -O-C₁₋₄ alkyl, -C(O)-NR₆ₐR_{6b} or -NR_{6c}-C(O)-C₁₋₄ alkyl,
R₅ₐ, R_{5b}, R₆ₐ, R_{6b}, R_{6c}, R₇, R₈ₐ and R_{8b} are each independently selected from H and C₁₋₆ alkyl.

In another aspect, the present invention provides a compound represented by formula (I) or a pharmaceutically acceptable salt, solvate, polymorph, tautomer, metabolite, or prodrug thereof, 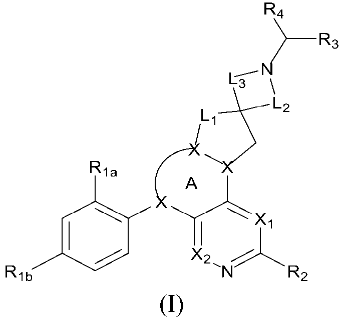 wherein,
Ring A is a 5- to 8-membered nitrogen-containing heterocyclic ring or nitrogen-containing heteroaromatic ring, each X is independently C or N,
R₁ₐ is -C(=O)-NR₂ₐR_{2b}, 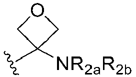 or 5-6-membered heteroaryl, the heteroaryl contains 1-3 nitrogen atoms, and optionally contains carbonyl, and the phenyl and heteroaryl are optionally substituted by 3-6-membered cycloalkyl or C₁₋₄ alkyl,
R₂ₐ and R_{2b} are each independently H or C₁₋₄ alkyl, the alkyl is optionally substituted with -OH or -NH₂,
R_{1b} is F or Cl,
R₂ is selected from H, halogen, C₁₋₄ alkyl, -O-C₁₋₄ alkyl and -NR₃ₐR_{3b},
R₃ₐ and R_{3b} are each independently H or C₁₋₄ alkyl,
X₁ and X₂ are each independently N or CH,
L₁ is -CH₂-, -CH₂-CH₂- or -CH₂-CH₂-CH₂-,
L₂ and L₃ are each independently -CH₂- or -CH₂-CH₂-,
R₄ is C₁₋₆ alkyl, 3-8-membered cycloalkyl or 3-8-membered heterocycloalkyl,
R₃ is -C₁₋₆ alkylene -NR₄ₐR_{4b}, -C₁₋₆ alkylene -C(O)-NR₅ₐR_{5b}, -C₁₋₆ alkylene -OH, or -C₁₋₆ alkylene -NR₇-C(O)-O-C₁₋₄ alkylene -O-C(O)-C₁₋₄ alkyl, wherein the alkyl or alkylene are optionally substituted with halogen, -CN, -OH or -O-C₁₋₄ alkyl,
R₄ₐ and R_{4b} are each independently selected from H, C₁₋₆ alkyl, -C(O)-C₁₋₄ alkyl, -C(O)-O-C₁₋₄ alkyl and -C(O)-NR₈ₐR_{8b}, the C₁₋₆ alkyl is optionally substituted with halogen, -CN, -OH, -S(O)₂-C₁₋₄ alkyl, -O-C₁₋₄ alkyl, -C(O)-NR₆ₐR_{6b} or -NR_{6c}-C(O)-C₁₋₄ alkyl,
R₅ₐ, R_{5b}, R₆ₐ, R_{6b}, R_{6c}, R₇, R₈ₐ and R_{8b} are each independently selected from H and C₁₋₆ alkyl.

In some embodiments, 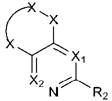 is 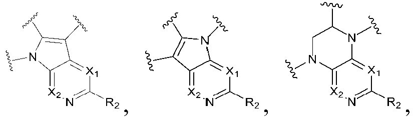 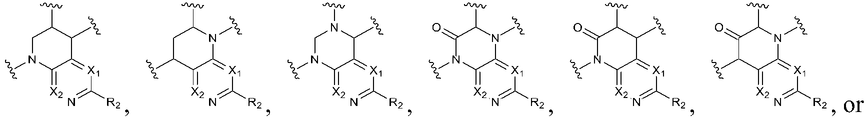 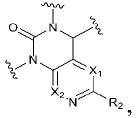 preferably

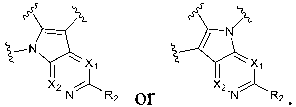

In some embodiments, R₁ₐ is -C(=O)-NR₂ₐR_{2b}, R₂ₐ and R_{2b} are each independently H or C₁₋₄ alkyl.

In some embodiments, R₁ₐ is

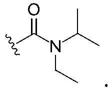

In some embodiments, R_{1b} is F.

In some embodiments, R₄ is isopropyl.

In some embodiments, R₂ is H or C₁₋₄ alkyl, preferably H.

In some embodiments, R₃ is -C₁₋₆ alkylene -NR₄ₐR_{4b}, R₄ₐ and R_{4b} are each independently selected from C₁₋₆ alkyl, the C₁₋₆ alkyl is optionally substituted with -O-C₁₋₄ alkyl.

In some embodiments, R₃ is -C₁₋₆ alkylene -N(C₁₋₆ alkyl )-C₁₋₆ alkylene -O-C₁₋₄ alkyl.

In some embodiments, R₃ is

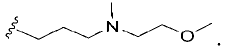

In some embodiments, the present invention provides the following compound or a pharmaceutically acceptable salt, solvate, polymorph, tautomer, metabolite, or prodrug thereof

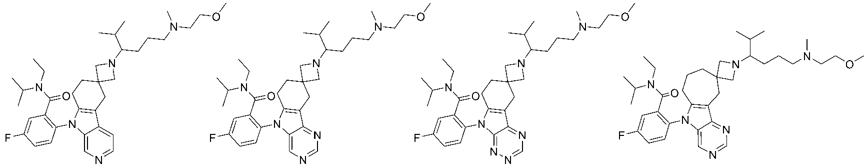

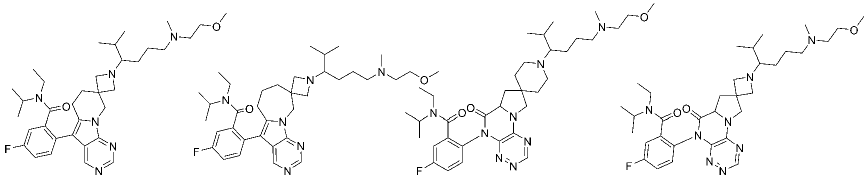

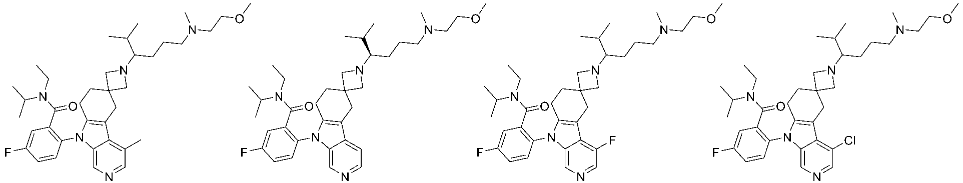

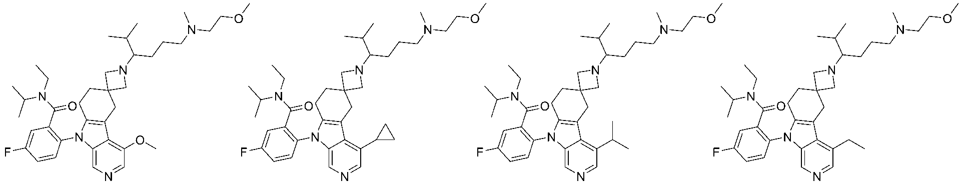

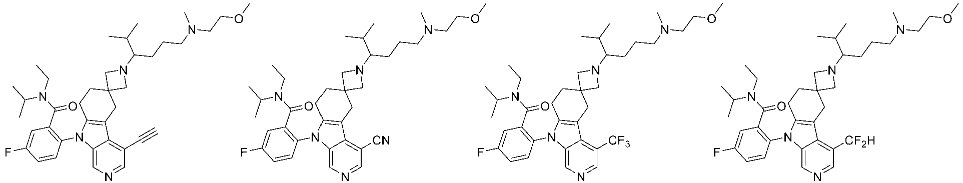

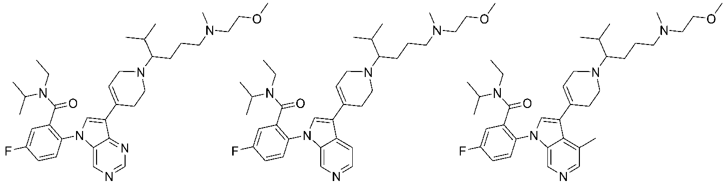

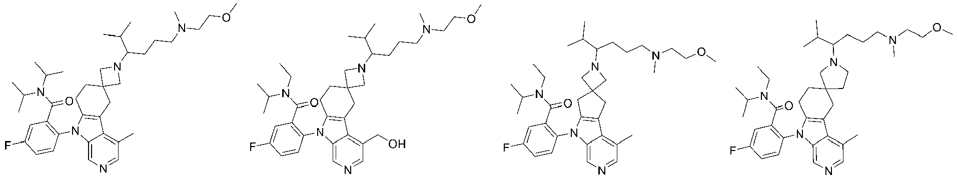

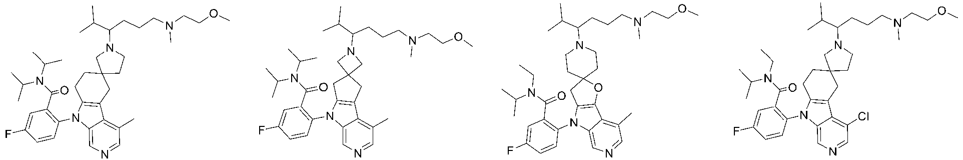

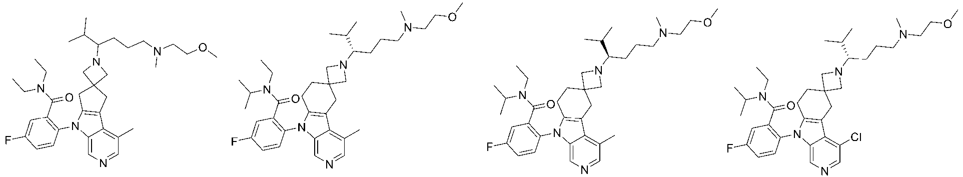

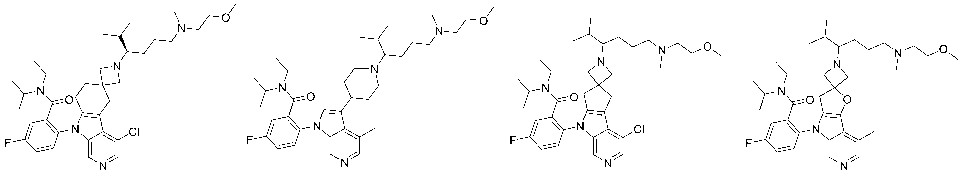

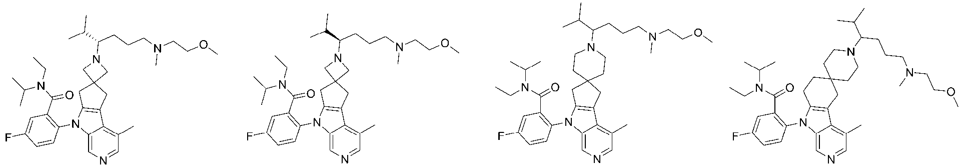

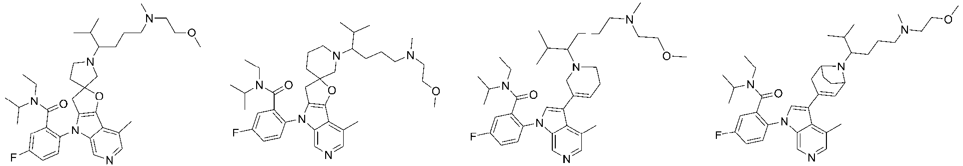

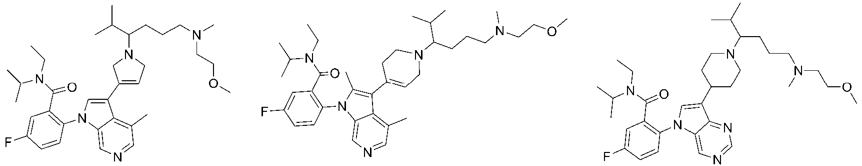

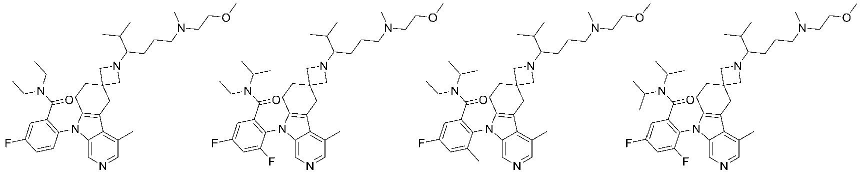

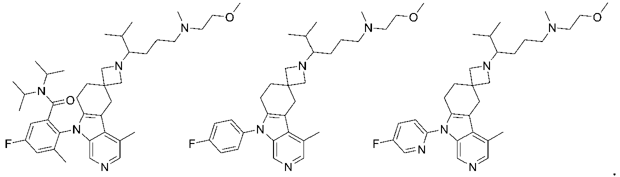

In another aspect, the present invention provides a pharmaceutical composition comprising a compound of the present invention or a pharmaceutically acceptable salt, solvate, polymorph, or isomer thereof, and optionally comprising a pharmaceutically acceptable carrier.

In another aspect, the present invention provides a method for treating a disease associated with MLL activity, comprising administering to a subject a therapeutically effective amount of a compound of the present invention or a pharmaceutically acceptable salt, solvate, polymorph, tautomer, metabolite, or prodrug thereof, or a pharmaceutical composition of the present invention; In some embodiments, The disease associated with MLL activity is cancer, preferably acute leukemia (including MLL acute leukemia, MLL partial tandem duplication acute leukemia, NPM-mutant acute leukemia, MOZ acute leukemia, NUP98 acute leukemia, and CALM acute leukemia), chronic lymphocytic leukemia, chronic myeloid leukemia, myelodysplastic syndrome, polycythemia vera, malignant lymphoma (including B-cell lymphoma), myeloma (including multiple myeloma), brain tumor, head and neck cancer, esophageal cancer, thyroid cancer, small cell lung cancer, non-small cell lung cancer, breast cancer, gastric cancer, gallbladder and bile duct cancer, liver cancer, hepatocellular carcinoma, pancreatic cancer, colon cancer, rectal cancer, anal cancer, choriocarcinoma, endometrial cancer, cervical cancer, ovarian cancer, bladder cancer, urothelial carcinoma, renal cancer, renal cell carcinoma, prostate cancer, testicular tumor, testicular germ cell tumor, ovarian germ cell tumor, Wilms tumor, malignant melanoma, neuroblastoma, osteosarcoma, Ewing's sarcoma, chondrosarcoma, soft tissue sarcoma, or skin cancer.

The compound of the present invention or a pharmaceutically acceptable salt, solvate, polymorph, tautomer, metabolite, or prodrug thereof, or the pharmaceutical composition of the present invention, is used in combination with at least one different agent, wherein the different agent is selected from at least one agent of antineoplastic alkylating agents, antineoplastic antibiotics, plant-derived antineoplastic agents, antineoplastic platinum coordination compounds, antineoplastic camptothecin derivatives, antineoplastic tyrosine kinase inhibitors, antineoplastic serine/threonine kinase inhibitors, antineoplastic phospholipid kinase inhibitors, antineoplastic monoclonal antibodies, interferons, biological response modifiers, hormonal agents, angiogenesis inhibitors, immune checkpoint inhibitors, epigenetics-related molecular inhibitors, protein post-translational modification inhibitors, proteasome inhibitors, and other antineoplastic drugs..

In another aspect, the present invention provides use of the compound of the present invention or a pharmaceutically acceptable salt, solvate, polymorph, tautomer, metabolite, or prodrug thereof, or the pharmaceutical composition of the present invention in the manufacture of a medicament for the treatment of a disease associated with MLL activity; In some embodiments, the disease associated with MLL activity is cancer, preferably Acute leukemia (including MLL acute leukemia, MLL partial tandem duplication acute leukemia, NPM mutant acute leukemia, MOZ acute leukemia, NUP98 acute leukemia, and CALM acute leukemia), Chronic lymphocytic leukemia, chronic myeloid leukemia, myelodysplastic syndrome, polycythemia vera, malignant lymphoma (including B-cell lymphoma), myeloma (including multiple myeloma), Brain tumor, head and neck cancer, esophageal cancer, thyroid cancer, small cell lung cancer, non-small cell lung cancer, breast cancer, gastric cancer, gallbladder and bile duct cancer, liver cancer, hepatocellular carcinoma, Pancreatic cancer, colon cancer, rectal cancer, anal cancer, choriocarcinoma, endometrial cancer, cervical cancer, ovarian cancer, bladder cancer, urothelial carcinoma, Kidney cancer, renal cell carcinoma, prostate cancer, testicular tumor, testicular germ cell tumor, ovarian germ cell tumor, Wilms tumor, malignant melanoma, neuroblastoma, osteosarcoma, Ewing's sarcoma, chondrosarcoma, soft tissue sarcoma, or skin cancer.

### Detailed Description of the Invention

Exemplary embodiments utilizing the principles of the present invention are set forth in the detailed description below. The features and advantages of the present invention may be better understood by reference to the following disclosure.

It should be understood that the scope of protection of various aspects of the present invention is defined by the claims, and that methods and structures within the scope of these claims, as well as their equivalent methods and structures, are within the scope covered by these claims.

Unless otherwise defined, all technical and scientific terms used herein have the same meaning as commonly understood by those skilled in the art to which the claimed subject matter pertains. Unless otherwise indicated, all patents, patent applications, and published materials cited throughout this specification are hereby incorporated by reference in their entirety.

It should be understood that both the foregoing brief description and the following detailed description are exemplary and explanatory and are not restrictive of any subject matter of the present invention. Unless specifically stated otherwise, the singular forms also include the plural. Unless otherwise indicated, the use of "or" means "and/or." Furthermore, the use of the term "including," as well as other forms such as "comprising," "containing," and "having," is not limiting.

### Certain Chemical Terminology

The terms "optional" or "optionally" (as used herein) mean that the subsequently described event or circumstance may or may not occur, and the description includes instances where the event or circumstance occurs and instances where it does not. For example, "optionally substituted alkyl" means "unsubstituted alkyl" or "substituted alkyl". Moreover, an optionally substituted group may be unsubstituted (e.g., -CH₂CH₃), fully substituted (e.g., -CF₂CF₃), monosubstituted (e.g., -CH₂CH₂F), or substituted at any level between monosubstitution and full substitution (e.g., -CH₂CHF₂, -CF₂CH₃, -CFHCHF₂, etc.). Those skilled in the art will appreciate that for any group containing one or more substituents, no substitution or substitution pattern that is sterically impractical and/or cannot be synthesized is introduced.

Unless otherwise specified, conventional methods within the scope of the art, such as mass spectrometry, nuclear magnetic resonance, high-performance liquid chromatography, infrared and ultraviolet/visible spectroscopy, and pharmacological methods, are employed. Unless specific definitions are provided, the relevant terms, experimental procedures, and techniques in analytical chemistry, organic synthetic chemistry, and medicinal and pharmaceutical chemistry are those known in the art. Standard techniques may be used in chemical synthesis, chemical analysis, drug preparation, formulation and delivery, and patient treatment. For example, reactions and purifications may be carried out using manufacturer's instructions for kits, or in a manner well known in the art or as described in the present invention. Typically, the above techniques and methods can be implemented according to conventional methods well known in the art, based on descriptions in the various general and more specific references cited and discussed in this specification. In this specification, groups and substituents thereof may be selected by those skilled in the art to provide stable structural moieties and compounds.

When a substituent is described by a conventional chemical formula written from left to right, the substituent equally includes the chemically equivalent substituent obtained by writing the structural formula from right to left. For example, -CH₂O- is equivalent to -OCH₂-.

As used herein, the terms "group" or "chemical group" refer to a specific portion or functional group of a molecule. A chemical group is often considered as a chemical entity embedded in or attached to a molecule.

Some chemical groups named herein may be denoted by a shorthand notation indicating the total number of carbon atoms. For example, "C₁-C₆ alkyl" describes an alkyl group, as defined below, having a total of 1 to 6 carbon atoms. The total number of carbon atoms indicated in the shorthand notation does not include carbon atoms from possible substituents.

The term "halogen", "halo", or "halide" refers to bromine, chlorine, fluorine, or iodine.

As used herein, the terms "aromatic", "aromatic ring", "aromatic group", "aromaticity", "aromatic ring system" refer to a planar cyclic moiety of one or more rings having a delocalized conjugated π-electron system containing 4n + 2 electrons, wherein n is an integer. An aromatic ring can be formed from 5, 6, 7, 8, 9, or more than 9 atoms. Aromatic compounds may be optionally substituted and may be monocyclic or polycyclic with fused rings. The term aromatic compounds includes all carbocyclic rings (e.g., benzene rings) and rings containing one or more heteroatoms (e.g., pyridine).

As used herein, the term "heteroatom" or "hetero", used alone or as part of another group, refers to atoms other than carbon and hydrogen. Heteroatoms are independently selected from oxygen, nitrogen, sulfur, phosphorus, silicon, selenium, and tin, but are not limited to these atoms. In embodiments where two or more heteroatoms are present, said two or more heteroatoms may be the same as each other, or some or all of said two or more heteroatoms may be different from each other.

As used herein, the term "fused" or "fused ring", used alone or in combination, refers to a cyclic structure in which two or more rings share one or more bonds.

As used herein, the term "spiro" or "spiro ring", used alone or in combination, refers to a cyclic structure in which two or more rings share one or more atoms.

As used herein, the term "alkyl", used alone or as part of another group (e.g., monoalkylamino), refers to an optionally substituted straight-chain or optionally substituted branched-chain monovalent saturated hydrocarbon group having 1 to 12 carbon atoms, preferably 1 to 8 carbon atoms, more preferably 1 to 6 carbon atoms, which is attached to the rest of the molecule by a single bond, such as methyl, ethyl, n-propyl, isopropyl, n-butyl, isobutyl, sec-butyl, tert-butyl, n-pentyl, n-hexyl, n-heptyl, 2-methylhexyl, 3-methylhexyl, n-octyl, n-nonyl, n-decyl, and the like.

As used herein, the term "alkylene" refers to an optionally substituted straight-chain or optionally substituted branched-chain divalent saturated hydrocarbon group having 1 to 12 carbon atoms, preferably 1 to 8 carbon atoms, more preferably 1 to 6 carbon atoms.

The term "aryl" refers to an all-carbon monocyclic or fused ring having a fully conjugated π-electron system, containing 6 to 14 carbon atoms, preferably 6 to 12 carbon atoms, most preferably 6 carbon atoms. Aryl may be unsubstituted or substituted with one or more substituents, examples of which include, but are not limited to, alkyl, alkoxy, aryl, aralkyl, amino, halogen, hydroxy, sulfonyl, sulfinyl, phosphoryl, and heterocyclyl. Non-limiting examples of unsubstituted aryl include, but are not limited to, phenyl, naphthyl, and anthracenyl.

The term "heteroaryl" refers to a monocyclic or fused ring of 5 to 12 ring atoms, having 5, 6, 7, 8, 9, 10, 11 or 12 ring atoms, which contains 1, 2, 3 or 4 ring atoms selected from N, O, and S, with the remaining ring atoms being C, and having a fully conjugated π-electron system. Heteroaryl may be unsubstituted or substituted, wherein the substituents include, but are not limited to, alkyl, alkoxy, aryl, aralkyl, amino, halogen, hydroxy, cyano, nitro, carbonyl, and heterocyclyl. Non-limiting examples of unsubstituted heteroaryl include, but are not limited to, pyrrolyl, furanyl, thienyl, imidazolyl, oxazolyl, pyrazolyl, pyridinyl, pyrimidinyl, pyrazinyl, quinolinyl, isoquinolinyl, tetrazolyl, triazinyl.

As used herein, the term "cycloalkyl," used alone or in combination, refers to a stable monovalent non-aromatic monocyclic or polycyclic hydrocarbon group containing only carbon and hydrogen atoms, which may include fused, spiro, or bridged ring systems, having 3 to 15 ring-forming carbon atoms, preferably 3 to 10 ring-forming carbon atoms, more preferably 3 to 8 ring-forming carbon atoms, which may be saturated or unsaturated, and is attached to the rest of the molecule by a single bond. Non-limiting examples of "cycloalkyl" include, but are not limited to, cyclopropyl, cyclobutyl, cyclopentyl, cyclohexyl, and cycloheptyl.

As used herein, the terms "heterocyclyl", "heterocycloalkyl", and "heterocycle", used alone or as part of another group, refer to a stable 3- to 18-membered monovalent non-aromatic ring containing 2 to 12 carbon atoms and 1 to 6 heteroatoms selected from nitrogen, oxygen, and sulfur. Unless otherwise specified, a heterocyclyl group can be a monocyclic, bicyclic, tricyclic, or tetracyclic ring system, which may include fused, spiro, or bridged ring systems. The nitrogen, carbon, or sulfur atoms of the heterocyclyl group may be optionally oxidized, the nitrogen atom may be optionally quaternized, and the heterocyclyl group may be partially or fully saturated. The heterocyclyl group can be attached to the rest of the molecule via a single bond from a ring carbon or heteroatom. A heterocyclyl group containing fused rings may include one or more aromatic or heteroaromatic rings, provided that the point of attachment to the rest of the molecule is on a non-aromatic ring atom. For the purposes of this application, the heterocyclyl group is preferably a stable 4- to 11-membered monovalent non-aromatic monocyclic or bicyclic ring containing 1 to 3 heteroatoms selected from nitrogen, oxygen, and sulfur, more preferably a stable 4- to 8-membered monovalent non-aromatic monocyclic ring containing 1 to 3 heteroatoms selected from nitrogen, oxygen, and sulfur. Non-limiting examples of heterocyclyl groups include azepanyl, azetidinyl, decahydroisoquinolinyl, dihydrofuranyl, dihydroindolyl, dioxolanyl, 1,1-dioxothiomorpholinyl, imidazolidinyl, imidazolinyl, isothiazolidinyl, isoxazolidinyl, morpholinyl, octahydroindolyl, octahydroisoindolyl, oxazinyl, piperazinyl, piperidinyl, 4-piperidonyl, pyranyl, pyrazolidinyl, pyrrolidinyl, quinolizinyl, quinuclidinyl, tetrahydrofuranyl, tetrahydropyranyl, and the like.

As used herein, the term "polymorph" or "polymorphism" means that the compound of the present invention has multiple crystal lattice forms. Some compounds of the present invention may have more than one crystalline form, and the present invention encompasses all polymorphic forms or mixtures thereof.

Intermediate compounds of the compounds of the present invention and their polymorphs are also within the scope of the present invention.

Unless otherwise specified, the olefinic double bonds contained in the compounds of the present invention include both E and Z isomers.

The compounds of the present invention include those having one or more isotopic substitutions, and reference to a particular element within its scope includes all isotopes of that element. For example, reference to hydrogen within its scope includes ¹H, ²H(D) and ³H(T). Similarly, reference to carbon and oxygen within their scopes include ¹²C, ¹³C and ¹⁴C, as well as ¹⁶O and ¹⁸O, respectively.

It should be understood that the compounds of the present invention may contain asymmetric centers. These asymmetric centers can independently be in the R or S configuration. Some compounds of the present invention may also exhibit cis-trans isomerism, as will be apparent to those skilled in the art. It should be understood that the compounds of the present invention include their individual geometric isomers and stereoisomers, as well as mixtures thereof, including racemic mixtures. These isomers can be separated from their mixtures by performing or modifying known methods, such as chromatographic techniques and recrystallization techniques, or can be prepared individually from suitable isomers of their intermediates.

As used herein, the term "pharmaceutically acceptable salts" includes both acid addition salts and base addition salts.

"Pharmaceutically acceptable acid addition salts" refer to those salts that retain the biological effectiveness and properties of the free base of the compound, are not biologically or otherwise undesirable, and are formed with inorganic acids such as, but not limited to, hydrochloric acid, hydrobromic acid, sulfuric acid, nitric acid, phosphoric acid, and the like, or with organic acids such as, but not limited to, acetic acid, 2,2-dichloroacetic acid, adipic acid, alginic acid, ascorbic acid, aspartic acid, benzenesulfonic acid, benzoic acid, capric acid, caproic acid, carbonic acid, cinnamic acid, citric acid, and the like. "Pharmaceutically acceptable base addition salts" refer to those salts that retain the biological effectiveness and properties of the free acid of the compound, and are not biologically or otherwise undesirable. These salts are prepared by reacting the free acid with an inorganic base or an organic base. Salts formed with inorganic bases include, but are not limited to, sodium salts, potassium salts, lithium salts, ammonium salts, calcium salts, magnesium salts, iron salts, zinc salts, copper salts, manganese salts, aluminum salts, and the like. Preferred inorganic salts are ammonium salts, sodium salts, potassium salts, calcium salts, and manganese salts.

Organic bases that form salts include, but are not limited to, primary amines, secondary amines, tertiary amines, cyclic amines, and the like, such as ammonia, isopropylamine, trimethylamine, diethylamine, triethylamine, tripropylamine, ethanolamine, diethanolamine, ethylenediamine, dicyclohexylamine, purine, piperazine, piperidine, choline, and caffeine. Particularly preferred organic bases are isopropylamine, diethylamine, ethanolamine, trimethylamine, dicyclohexylamine, choline, and caffeine.

Crystallization often produces solvates of the compounds of the present invention. As used herein, the term "solvate" refers to an aggregate composed of one or more molecules of the compound of the present invention and one or more solvent molecules.

The solvent may be water, in which case the solvate is a hydrate. Alternatively, it may be an organic solvent. Therefore, the compounds of the present invention may exist as hydrates, including monohydrates, dihydrates, hemihydrates, trihydrates, tetrahydrates, and the like, as well as corresponding solvated forms. The compounds of the present invention may be true solvates; however, in other instances, they may merely retain adventitious water or a mixture of water and some other solvents. The compounds of the present invention may be reacted in a solvent, or precipitated or crystallized from a solvent. Solvates of the compounds of the present invention are also within the scope of the present invention.

As used herein, the term "pharmaceutical composition" refers to a formulation comprising a compound of the present invention and a medium generally accepted in the art for delivering biologically active compounds to mammals (e.g., humans). Such a medium includes all pharmaceutically acceptable carriers.

As used herein, the term "acceptable" with respect to a formulation, composition, or ingredient means having no sustained detrimental effect on the general health of the subject being treated.

As used herein, the term "pharmaceutically acceptable" refers to a substance (such as a carrier or diluent) that does not affect the biological activity or properties of the compound of the present invention and is relatively non-toxic, i.e., the substance can be administered to an individual without causing adverse biological reactions or interacting in an undesirable manner with any component contained in the composition.

"Pharmaceutically acceptable carrier" includes, but is not limited to, adjuvants, vehicles, excipients, auxiliary agents, deodorants, diluents, preservatives, dyes/colorants, flavor enhancers, surfactants and wetting agents, dispersing agents, suspending agents, stabilizers, isotonic agents, solvents, or emulsifiers that have been approved by the relevant government administrative department for use in humans and domestic animals.

As used herein, the terms "subject," "patient," "object," or "individual" refer to an individual suffering from a disease, disorder, condition, or the like, including mammals and non-mammals. Examples of mammals include, but are not limited to, any member of the class Mammalia: humans; non-human primates (e.g., chimpanzees and other apes and monkeys); domestic animals such as cattle, horses, sheep, goats, and pigs; domesticated animals such as rabbits, dogs, and cats; laboratory animals including rodents such as rats, mice, and guinea pigs, and the like. Examples of non-mammals include, but are not limited to, birds, fish, and the like. In one embodiment of the methods and compositions provided herein, the mammal is a human.

As used herein, the term "treatment" refers to the treatment of a disease or condition in a mammal, particularly a human, including:
(i) preventing the disease or condition from occurring in a mammal, particularly a mammal that has been previously exposed to a disease or condition but has not yet been diagnosed with the disease or condition;
(ii) inhibiting the disease or condition, i.e., arresting its development;
(iii) alleviating the disease or condition, i.e., causing regression of the disease or condition;
(iv) alleviating the symptoms caused by the disease or condition.

As used herein, the terms "disease" and "disorder" may be used interchangeably, or may have different meanings, because certain specific diseases or disorders have no known causative agent (hence the cause is not yet understood) and thus cannot be recognized as diseases but rather as unwanted conditions or syndromes, which have more or less specific symptoms that have been confirmed by clinical researchers.

As used herein, the terms "effective amount," "therapeutically effective amount," or "pharmaceutically effective amount" refer to an amount of at least one agent or compound that, upon administration, is sufficient to alleviate, to some extent, one or more symptoms of the disease or condition being treated. The result may be the reduction and/or alleviation of signs, symptoms, or causes, or any other desired change in a biological system. For example, an "effective amount" for therapeutic use is the amount of a composition comprising a compound disclosed herein that provides a clinically significant alleviation of symptoms of the condition. Techniques such as dose escalation studies may be used to determine the effective amount suitable for any individual case.

As used herein, the terms "administering," "administration," and the like refer to methods that are capable of delivering a compound or composition to the desired site of biological action. These methods include, but are not limited to, oral routes, intraduodenal routes, parenteral injection (including intravenous, subcutaneous, intraperitoneal, intramuscular, intra-arterial injection or infusion), topical administration, and rectal administration. In a preferred embodiment, the compounds and compositions discussed herein are administered orally.

### Preparation of the compounds of the present invention

The following specific examples are intended to enable those skilled in the art to more clearly understand and practice the present invention. They should not be considered as limiting the scope of the invention, but rather as illustrative and representative examples of the invention. Those skilled in the art will appreciate that there are other synthetic routes to form the compounds of the present invention, and the examples provided below are non-limiting.

All operations involving starting materials that are easily oxidized or hydrolyzed are carried out under nitrogen protection. Unless otherwise specified, the starting materials used in the present invention are directly purchased from commercial sources and used without further purification.

Column chromatography was performed using silica gel (200 - 300 mesh) produced by Qingdao Chemical Co., Ltd. Thin-layer chromatography (TLC) was carried out on precoated plates (silica gel 60 PF₂₅₄, 0.25 mm) manufactured by E. Merck. Separation of chiral compounds and determination of enantiomeric excess (ee) were performed using Agilent LC 1200 series (column: CHIRALPAK AD-H, Ø 4.6 x 250 mm, 5 µm, 30 °C). Nuclear magnetic resonance (NMR) spectra were measured using a Varian VNMRS-400 NMR spectrometer; liquid chromatography-mass spectrometry (LC/MS) was performed using a FINNIGAN Thermo LCQ Advantage MAX, Agilent LC 1200 series (column: Waters Symmetry C18, Ø4.6 x 50 mm, 5 µm, 35 °C), with ESI(+) ion mode.

### Experimental Section

### Intermediate 1: 6-((2-methoxyethyl)(methylamino)-2-methylhexan-3-one

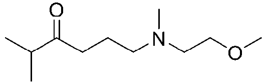

6-((2-Methoxyethyl)(methyl)amino)-2-methylhexan-3-one was synthesized according to the same method as that for Intermediate 162 in patent WO2021121327.

### Intermediate 2: 2-(7',8'-dihydrospiro[azetidine-3,6'-pyrido[3,4-b]indol]-9'(5'H)-yl)-N-eth yl-5-fluoro-N-isopropylbenzamide

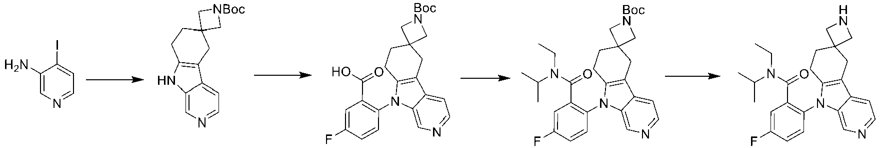

### Step 1: tert-butyl 5',7',8',9'-tetrahydrospiro[azetidine-3,6'-pyrido[3,4-b]indol]-1-carboxyl ate

Under nitrogen protection, 4-iodopyridin-3-amine (880 mg), *tert-butyl* 7-oxo-2-azaspiro [3.5]nonane-2-carboxylate (956 mg), palladium acetate (90 mg) and 1,4-diazabicyclo [2.2.2]octane (1.35 g) were added to N,N-dimethylformamide (10 mL), and the mixture was heated to 120 °C and reacted for 8 hours. The reaction solution was cooled to room temperature and poured into water (50 mL), and the mixture was extracted three times with ethyl acetate (20 mL). The extracts were combined and washed sequentially with water and brine, dried over anhydrous sodium sulfate, and concentrated under reduced pressure. The residue was separated through silica gel column chromatography (petroleum ether/ethyl acetate, 3: 1) to obtain the target product (790 mg). MS m/z [LC-MS]: 314.19 [M+1].

### Step 2: 2-(1-(tert-butoxycarbonyl)-7',8'-dihydrospiro[azetidine-3,6'-pyrido[3,4-b]indol]-9 '(5'H)-yl)-5-fluorobenzoic acid

Under nitrogen protection, *tert-butyl* 5',7',8',9'-tetrahydrospiro[azetidine-3,6'-pyrido[3,4-b] indol]-1-carboxylate (628 mg), 5-fluoro-2-iodobenzoic acid (638 mg), *(1S,2S)-N*¹,*N*²-dimethylcyclohexane-1,2-diamine (28 mg), copper(I) iodide (38 mg) and potassium phosphate (1.27 g) were added to 1,4-dioxane (10 mL), and the mixture was heated to 100 °C and reacted for 8 hours. The reaction solution was cooled to room temperature, poured into water (50 mL), and adjusted to pH 4-5 with 1 M hydrochloric acid. The mixture was filtered, and the solid was dried and separated through silica gel column chromatography (dichloromethane/methanol, 15:1) to obtain the target product (500 mg). MS m/z [LC-MS]: 451.20 [M+1].

### Step 3: tert-butyl 9'-(2-(ethyl(isopropyl)carbamoyl)-4-fluorophenyl)-5',7',8',9'-tetrahydro spiro[azetidine-3,6'-pyrido[3,4-b]indol]-1-carboxylate

2-(1-(*Tert*-butoxycarbonyl)-7',8'-dihydrospiro[azetidine-3,6'-pyrido[3,4-b]indol]-9'(5'*H*)-yl)-5-fluorobenzoic acid (450 mg), *N*-ethylpropan-2-amine (104 mg), diisopropylethylamine (387 mg) and 2-(7-azabenzotriazol)-N,N,N',N'-tetramethyluronium hexafluorophosphate (380 mg) were added to acetonitrile (5 mL), the mixture was stirred at room temperature for 2 hours and concentrated under reduced pressure to remove the solvent. The residue was dissolved in dichloromethane (20 mL), the solution was washed with water, and the organic phase was concentrated under reduced pressure and separated through silica gel column chromatography (petroleum ether/ethyl acetate, 2:1) to obtain the target product (500 mg). MS m/z [LC-MS]: 521.29 [M+1].

### Step 4: 2-(7',8'-dihydrospiro[azetidine-3,6'-pyrido[3,4-b]indol]-9'(5'H)-yl)-N-ethyl-5-fluo ro-N-isopropylbenzamide

*Tert-butyl* 9'-(2-(ethyl(isopropyl)carbamoyl)-4-fluorophenyl)-5',7',8',9'-tetrahydrospiro [azetidine-3,6'-pyrido[3,4-b]indol]-1-carboxylate (480 mg) was dissolved in dichloromethane (10 mL), trifluoroacetic acid (3 mL) was added, and the mixture was stirred at room temperature for 2 hours. The mixture was adjusted to pH 12-13 with 10% sodium hydroxide solution, the organic phase was separated, and the aqueous phase was extracted with dichloromethane. The organic phases were combined, washed sequentially with water and brine, dried over anhydrous sodium sulfate, and concentrated under reduced pressure to obtain the target compound (380 mg), which was used directly in the next step. MS m/z [LC-MS]: 421.24 [M+1].

### Intermediate 3: 2-(6',9'-dihydrospiro[azetidine-3,8'-pyrimido[5,4-b]indol]-5(7'H)-yl)-N-e thyl-5-fluoro-N-isopropylbenzamide

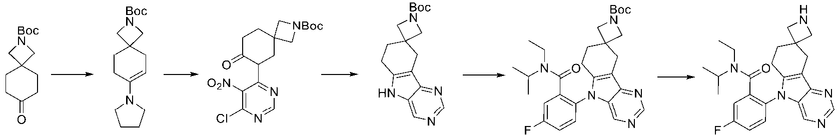

### Step 1: tert-butyl 7-(pyrrolidin-1-yl)-2-azaspiro[3.5]non-6-ene-2-carboxylate

Tert-butyl 7-oxo-2-azaspiro[3.5]nonane-2-carboxylate (480 mg), pyrrolidine (142 mg), and 3Å molecular sieve (1.0 g) were added to toluene (10 mL), and the mixture was stirred at room temperature for 8 hours. The molecular sieve was filtered off, and the solution was used directly in the next step. MS m/z [LC-MS]: 293.22 [M+1].

### Step 2: tert-butyl 6-(6-chloro-5-nitropyrimidin-4-yl)-7-oxo-2-azaspiro[3.5]nonane-2-car boxylate

The solution of *tert-butyl* 7-(pyrrolidin-1-yl)-2-azaspiro[3.5]non-6-ene-2-carboxylate in toluene prepared in step 1, 4,6-dichloro-5-nitropyrimidine (388 mg) and diisopropylethylamine (260 mg) were added to dichloromethane (10 mL), the mixture was stirred at room temperature for 8 hours, 1 M hydrochloric acid (10 mL) and ethanol (10 mL) were added, and the mixture was stirred for 2 hours. Dichloromethane (20 mL) was added, and the organic phase was separated, washed sequentially with water and brine, dried over anhydrous sodium sulfate, and concentrated under reduced pressure, and the residue separated through silica gel column chromatography (petroleum ether/ethyl acetate, 3:1) to obtain the target product (480 mg). MS m/z [LC-MS]: 397.13 [M+1].

### Step 3: tert-butyl 5',6',7',9'-tetrahydrospiro[azetidine-3,8'-pyrimido[5,4-b]indol]-1-carbo xylate

Tert-butyl 6-(6-chloro-5-nitropyrimidin-4-yl)-7-oxo-2-azaspiro[3.5]nonane-2-carboxylate (397 mg) and palladium on carbon (10%, 40 mg) were added to ethyl acetate (10 mL), the system was evacuated and then purged with hydrogen three times, and the mixture was reacted at room temperature for 4 hours. Palladium on carbon was filtered off, the filtrate was concentrated under reduced pressure and separated through silica gel column chromatography (petroleum ether/ethyl acetate, 2:1) to obtain the target compound (220 mg). MS m/z [LC-MS]: 315.18 [M+1].

### Step 4: tert-butyl 5'-(2-(ethyl(isopropyl)carbamoyl)-4-fluorophenyl)-5',6',7',9'-tetrahydro spiro[azetidine-3,8'-pyrimido[5,4-b]indol]-1-carboxylate

Under nitrogen protection, tert-butyl 5',6',7',9'-tetrahydrospiro[azetidine-3,8'-pyrimido [5,4-b]indol]-1-carboxylate(210 mg), *N*-ethyl-5-fluoro-2-iodo-*N*-isopropylbenzamide (335 mg), tris(dibenzylideneacetone)dipalladium (61 mg), 2-dicyclohexylphosphino-2',4',6'-triisopropyl biphenyl (32 mg) and sodium tert-butoxide (128 mg) were added to toluene (10 mL), the mixture was heated to 100°C and reacted for 8 hours, cooled to room temperature, and filtered, and the filtrate was concentrated under reduced pressure and separated through silica gel column chromatography (petroleum ether/ethyl acetate, 2:1) to obtain the target compound (180 mg). MS m/z [LC-MS]: 522.29 [M+1].

### Step 5: 2-(6',9'-dihydrospiro[azetidine-3,8'-pyrimido[5,4-b]indol]-5'(7'H)-yl)-N-ethyl-5-fl uoro-N-isopropylbenzamide

Following the method of Step 4 in Intermediate 2, tert-butyl 5'-(2-(ethyl(isopropyl) carbamoyl)-4-fluorophenyl)-5',6',7',9'-tetrahydrospiro[azetidine-3,8'-pyrimido[5,4-b]indol]-1-carboxylate was used as the starting material to obtain the target compound, which was used directly in the next step. MS m/z [LC-MS]: 422.24 [M+1].

### Intermediate 4: 2-(7',8'-dihydrospiro[azetidine-3,6'-[1,2,4]triazino[6,5-b]indol]-9'(5'H)-yl) -N-ethyl-5-fluoro-N-isopropylbenzamide

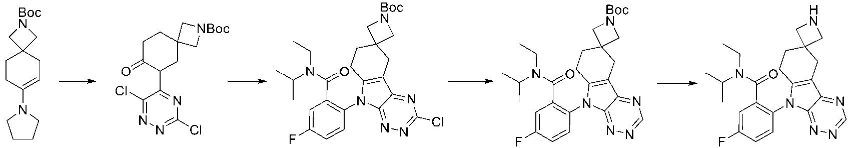

### Step 1: tert-butyl 6-(3,6-dichloro-1,2,4-triazin-5-yl)-7-oxo-2-azaspiro[3.5]nonane-2-carb oxylate

Following the method of Step 2 in Intermediate 3, 3,5,6-trichloro-1,2,4-triazine was used as the starting material to obtain the target product (540 mg). MS m/z [LC-MS]: 387.10 [M+1].

### Step 2: tert-butyl 3'-chloro-9'-(2-(ethyl(isopropyl)carbamoyl)-4-fluorophenyl)-5',7',8',9'-t etrahydrospiro[azetidine-3,6'-[1,2,4]triazino[6,5-b]indol]-1-carboxylate

Tert-butyl 6-(3,6-dichloro-1,2,4-triazin-5-yl)-7-oxo-2-azaspiro[3.5]nonane-2-carboxylate (387 mg), 2-amino-*N*-ethyl-5-fluoro-*N*-isopropylbenzamide(224 mg) and acetic acid (12 mg) were added to toluene (10 mL), the mixture was heated to 80°C and stirred for 10 hours, cooled to room temperature, and concentrated under reduced pressure, and the residue was separated through silica gel column chromatography (petroleum ether/ethyl acetate, 2:1) to obtain the target compound (260 mg). MS m/z [LC-MS]: 557.24 [M+1].

### Step 3: tert-butyl 9'-(2-(ethyl(isopropyl)carbamoyl)-4-fluorophenyl)-5',7',8',9'-tetrahydro spiro[azetidine-3,6'-[1,2,4]triazino[6,5-b]indol]-1-carboxylate

Following the method of Step 3 in Intermediate **3,** *tert*-butyl 3'-chloro-9'-(2-(ethyl (isopropyl)carbamoyl)-4-fluorophenyl)-5',7',8',9'-tetrahydrospiro[azetidine-3,6'-[1,2,4]triazino[6,5-*b*]indol]-1-carboxylate was used as the starting material to obtain the target compound (220 mg). MS m/z [LC-MS]: 523.28 [M+1].

### Step 4: 2-(7',8'-dihydrospiro[azetidine-3,6'-[1,2,4]triazino[6,5-b]indol]-9'(5'H)-yl)-N-ethy 1-5-fluoro-N-isopropylbenzamide

Following the method of Step 4 in Intermediate **2,** *tert*-butyl 9'-(2-(ethyl(isopropyl) carbamoyl)-4-fluorophenyl)-5',7',8',9'-tetrahydrospiro[azetidine-3,6'-[1,2,4]triazino[6,5-b]indol]-1-carboxylate was used as the starting material to obtain the target compound, which was used directly in the next step. MS m/z [LC-MS]: 423.23 [M+1].

### Intermediate 5: N-ethyl-5-fluoro-N-isopropyl-2-(4'-methyl-7',8'-dihydrospiro[azetidine-3, 6'-pyrido[3,4-b]indol]-9'(5'H)-yl)benzamide

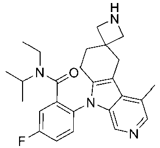

Following the synthetic method of Intermediate **2,** 4-iodo-5-methylpyridin-3-amine was used as the starting material to obtain the target product, which was used directly in the next step. MS m/z [LC-MS]: 435.26 [M+1].

### Intermediate 6: (R)-N¹-(2-methoxyethyl)-N¹,5-dimethylhexane-1,4-diamine hydrochloride

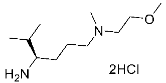

*(R)*-*N*¹-(2-methoxyethyl)-*N*¹,5-dimethylhexane-1,4-diamine hydrochloride was synthesized according to the same method as Intermediate **231** in patent WO2022262796.

### Intermediate 7: (R)-2-(6-((2-methoxyethyl)(methyl)amino)-2-methylhexan-3-yl)-2-azaspi ro[3.5]nonane-7-one

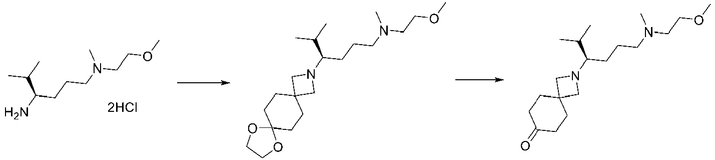

### Step 1: (R)-4-(8,11-dioxa-2-azaspiro[3.2.4⁷.2⁴]tridecan-2-yl)-N-(2-methoxyethyl)-N,5-di methylhexan-1-amine

(1,4-Dioxaspiro[4.5]decane-8,8-diyl)dimethanol (2.02 g) and diisopropylethylamine (5.16 g) were added to dry acetonitrile (50 mL), the mixture was cooled to -30°C, trifluoromethanesulfonic anhydride (6.21 g) was added dropwise, and the mixture was stirred at -30°C for 1 hour. Intermediate 5 (3.30 g) and diisopropylethylamine (5.16 g) were added, the mixture was refluxed for 24 horus, cooled to room temperature, and concentrated under reduced pressure to remove the solvent, saturated aqueous sodium bicarbonate solution (100 mL) was added, and the mixture was extracted with ethyl acetate (50 mL*3). The extract was washed sequentially with water and brine, dried over anhydrous sodium sulfate, and filtered, the filtrate concentrated under reduced pressure, and the residue was purified through silica gel column chromatography (dichloromethane/methanol, 15:1, with 2% concentrated ammonia water) to obtain the target compound (2.40 g). MS m/z [LC-MS]: 369.31 [M+1].

### Step 2: (R)-2-(6-((2-methoxyethyl)(methyl)amino)-2-methylhexan-3-yl)-2-azaspiro[3.5]n onane-7-one

*(R)*-4-(8,11-dioxa-2-azaspiro[3.2.4⁷.2⁴]tridecan-2-yl)-*N*-(2-methoxyethyl)-N,5-dimethylhexan-1-amine(1.85 g) and p-toluenesulfonic acid (2.15 g) were added to a mixed solvent of acetone-water (3:1, 20 mL), the mixture was stirred and refluxed for 16 hours, and cooled to room temperature, and 1 M sodium hydroxide solution was added to adjust pH to 12. The mixture was extracted with ethyl acetate (30 mL*3), the extract was washed sequentially with water and brine, dried over anhydrous sodium sulfate, and filtered, the filtrate concentrated under reduced pressure, and the residue was used directly in the next step. MS m/z [LC-MS]: 325.29 [M+1].

Intermediates **8~17, 21~35**, and **40-44** in the following table were prepared following the synthetic method of Intermediate **2,** using the corresponding starting materials.

| **Intermediate** | **Structure** | **MS[M+1]** | **Starting Material** |
|---|---|---|---|
| Intermediate **8** | 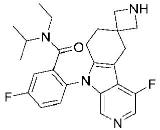 | 439.23 | 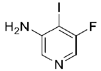 |
| Intermediate **9** | 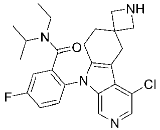 | 455.20 | 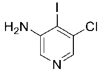 |
| Intermediate **10** | 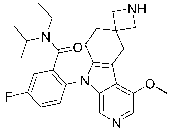 | 451.25 | 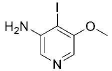 |
| Intermediate **11** | 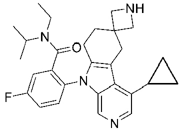 | 461.27 | 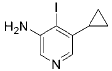 |
| Intermediate **12** | 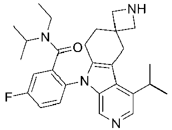 | 463.29 | 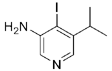 |
| Intermediate **13** | 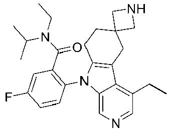 | 449.27 | 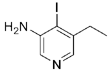 |
| Intermediate **14** | 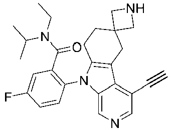 | 445.24 | 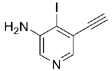 |
| Intermediate **15** | 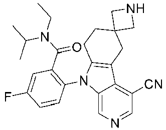 | 446.24 | 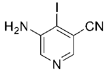 |
| Intermediate **16** | 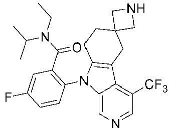 | 489.23 | 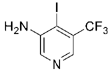 |
| Intermediate **17** | 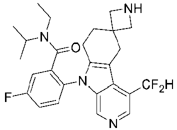 | 471.24 | 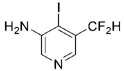 |
| Intermediate **21** | 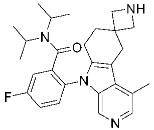 | 449.27 | 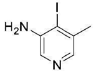 |
| | | | 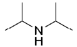 |
| Intermediate **22** | 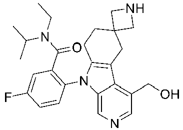 | 451.25 | 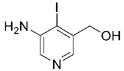 |
| Intermediate **23** | 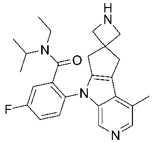 | 421.24 | 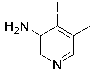 |
| | | | 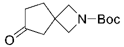 |
| Intermediate **24** | 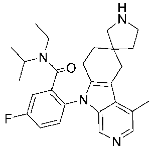 | 449.27 | 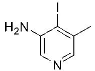 |
| | | | 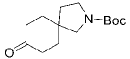 |
| Intermediate **25** | 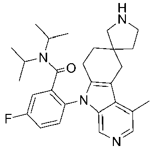 | 463.29 | 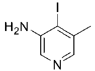 |
| | | | 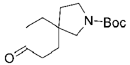 |
| | | | 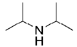 |
| Intermediate **26** | 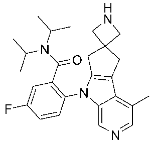 | 435.26 | 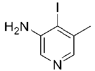 |
| | | | 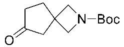 |
| | | | 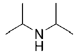 |
| Intermediate **27** | 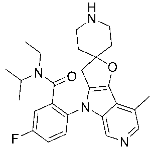 | 451.25 | 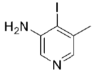 |
| | | | 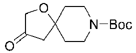 |
| Intermediate **28** | 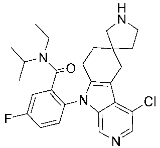 | 469.22 | 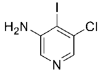 |
| | | | 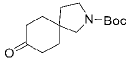 |
| Intermediate **29** | 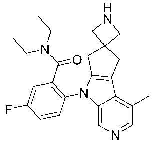 | 407.22 | 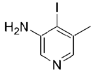 |
| | | | 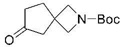 |
| | | | 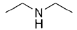 |
| Intermediate **30** | 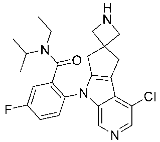 | 441.19 | 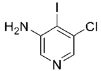 |
| | | | 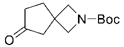 |
| Intermediate **31** | 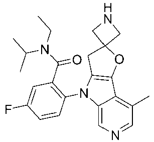 | 423.22 | 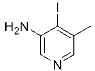 |
| | | | 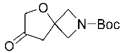 |
| Intermediate **32** | 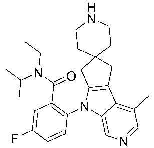 | 449.27 | 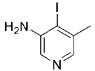 |
| | | | 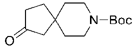 |
| Intermediate **33** | 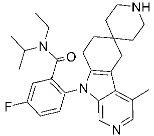 | 463.29 | 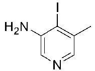 |
| | | | 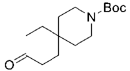 |
| Intermediate **34** | 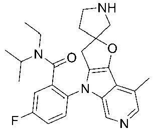 | 437.23 | 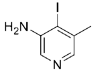 |
| | | | 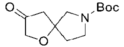 |
| Intermediate **35** | 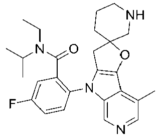 | 451.25 | 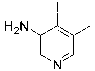 |
| | | | 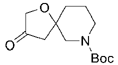 |
| Intermediate **40** | 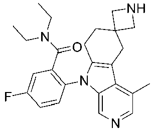 | 421.24 | 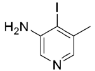 |
| | | | 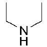 |
| Intermediate **41** | 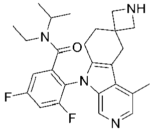 | 453.25 | 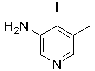 |
| | | | 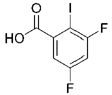 |
| Intermediate **42** | 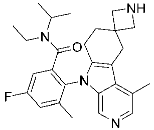 | 449.27 | 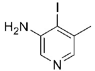 |
| | | | 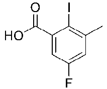 |
| Intermediate **43** | 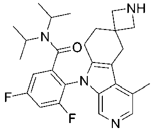 | 467.26 | 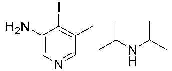 |
| | | | 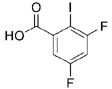 |
| Intermediate **44** | 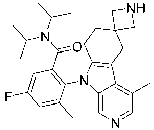 | 463.29 | 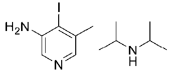 |
| | | | 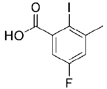 |

### Intermediate 18: N-ethyl-5- fluoro-N-isopropyl-2-(7-(1.2.3.6-tetrahydropyridin-4-yl)-5H-pyrrolo[3,2-d]pyrimidin-5-yl)benzamide

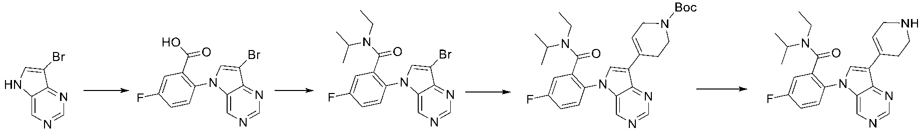

### Step 1: 2-(7-bromo-5H-pyrrolo[3,2-d]pyrimidin-5-yl)-5-fluorobenzoic acid

Under nitrogen protection, 7-bromo-5H-pyrrolo[3,2-d]pyrimidine (594 mg), 5-fluoro-2-iodobenzoic acid (798 mg), copper powder (191 mg) and potassium carbonate (414 mg) were added to N,N-dimethylformamide(6 mL), the reaction solution was heated to 100°C and stirred for 8 hours, cooled to room temperature, and filtered, the filtrate was poured into water (50 mL), and 1 M hydrochloric acid was used to adjust pH to 3-4. The mixture was filtered, and the filter cake was dried to obtain the target compound crude product (950 mg), which was used directly in the next step. MS m/z [LC-MS]: 335.98 [M+1].

### Step 2: 2-(7-bromo-5H-pyrrolo[3,2-d]pyrimidin-5-yl)-N-ethyl-5-fluoro-N-isopropylbenza mide

Following the method of step 3 in Intermediate 2, 2-(7-bromo-5H-pyrrolo [3,2-d]pyrimidin-5-yl)-5-fluorobenzoic acid was used as the starting material to obtain the target product. MS m/z [LC-MS]: 405.07 [M+1].

### Step 3: tert-butyl 4-(5-(2-(ethyl(isopropyl)carbamoyl)-4-fluorophenyl)-5H-pyrrolo[3,2-d]pyrimidin-7-yl)-3,6-dihydropyridine-1(2H)-carboxylate

Under nitrogen protection, 2-(7-bromo-5H-pyrrolo[3,2-d]pyrimidin-5-yl)-N-ethyl-5-fluoro-N-isopropylbenzamide (404 mg), *tert-butyl* 4-(4,4,5,5-tetramethyl-1,3,2- dioxaborolan-2-yl)-3,6-dihydropyridine-1(2H)-carboxylate (464 mg), Pd(dppf)Cl₂(73 mg) and potassium carbonate (414 mg) were added to a mixed solvent of 1,4-dioxane and water (8:1, 10 mL), and the reaction solution was heated to 100°C and stirred for 12 hours, cooled to room temperature, and concentrated under reduced pressure to remove the solvent. The residue was added to ethyl acetate (30 mL), the solution was washed with water and concentrated under reduced pressure, and the residue was purified through silica gel column chromatography (petroleum ether/ethyl acetate, 2:1) to obtain the target compound (310 mg). MS m/z [LC-MS]: 508.27 [M+1].

### Step 4: N-ethyl-5-fluoro-N-isopropyl-2-(7-(1,2,3,6-tetrahydropyridin-4-yl)-5H-pyrrolo[3, 2-d]pyrimidin-5-yl)benzamide

Following the method of Step 4 in Intermediate 2, tert-butyl 4-(5-(2-(ethyl(isopropyl) carbamoyl)-4-fluorophenyl)-5H-pyrrolo[3,2-d]pyrimidin-7-yl)-3,6-dihydropyridine-1(2H)-carboxylate was used as the starting material to obtain the target product. MS m/z [LC-MS]: 408.22 [M+1].

Intermediates **19~20** and **36~39** in the following table were prepared following the synthetic method of Intermediate **18,** using the corresponding starting materials.

| **Intermediate** | **Structure** | **MS[M+1]** | **Starting Material** |
|---|---|---|---|
| Intermediate **19** | 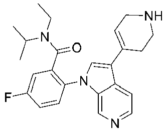 | 407.22 | 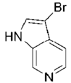 |
| Intermediate **20** | 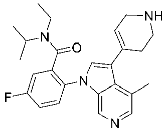 | 421.24 | 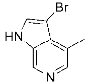 |
| Intermediate **36** | 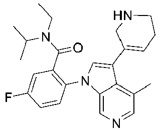 | 421.24 | 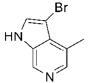 |
| | | | 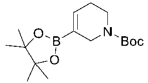 |
| Intermediate **37** | 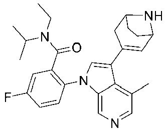 | 447.26 | 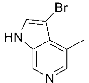 |
| | | | 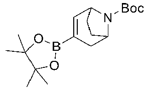 |
| Intermediate **38** | 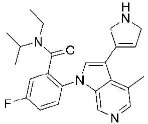 | 407.22 | 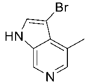 |
| | | | 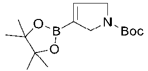 |
| Intermediate **39** | 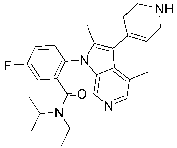 | 435.26 | 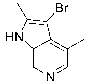 |

### Intermediate 45: 9'-(4-fluorophenyl)-5',7',8',9'-tetrahydrospiro[azetidine-3,6'-pyrido[3,4-b]indole]

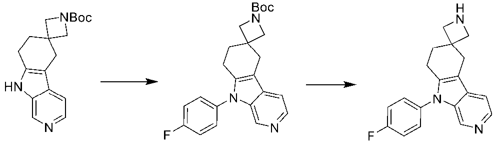

### Step 1: tert-butyl 9'-(4-fluorophenyl)-5',7',8',9'-tetrahydrospiro[azetidine-3,6'-pyrido[3,4-b]indole]-1-carboxylate

Under nitrogen protection, *tert-butyl* 5',7',8',9'-tetrahydrospiro[azetidine-3,6'-pyrido[3,4-b] indole]-1-carboxylate (628 mg), 1-fluoro-4-iodobenzene (888 mg), copper powder (64 mg) and cesium carbonate (1.96 g) were added to 1,4-dioxane (10 mL), the reaction solution was heated to 100 °C and reacted for 24 hours, cooled to room temperature, and filtered. The filtrate was evaporated to dryness, and the residue was separated through silica gel column chromatography (dichloromethane/methanol, 40:1) to obtain the target product (160 mg). MS m/z [LC-MS]: 408.21 [M+1].

### Step 2: 9'-(4-fluorophenyl)-5',7',8',9'-tetrahydrospiro[azetidine-3,6'-pyrido[3,4-b]indole]

*tert-butyl* 9'-(4-fluorophenyl)-5',7',8',9'-tetrahydrospiro[azetidine-3,6'-pyrido[3,4-b]indole]-1-carboxylate (160 mg) was dissolved in dichloromethane (2 mL), trifluoroacetic acid (1 mL) was added, and the mixture was stirred at room temperature for 2 hours. 10% Sodium hydroxide solution was used to adjust to pH 12-13, the organic phase was separated, the aqueous phase was extracted with dichloromethane, and the organic phases were combined, washed sequentially with water and brine, dried over anhydrous sodium sulfate, and concentrated under reduced pressure to obtain the target compound (110 mg), which was used directly in the next step. MS m/z [LC-MS]: 308.16 [M+1].

### Intermediate 46: 9'-(5-fluoropyridin-2-yl)-5',7',8',9'-tetrahydrospiro[azetidine-3,6'-pyrido [3,4-b]indole]

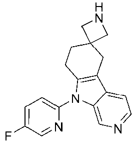

Following the synthetic method of Intermediate 45, 2-bromo-5-fluoropyridine was used as the starting material to obtain the target product, which was used directly in the next step. MS m/z [LC-MS]: 309.15 [M+1].

### Example 1: N-ethyl-5-fluoro-N-isopropyl-2-(1-(6-((2-methoxyethyl)(methyl)amino)-2-m ethylhexan-3-yl)-7',8'-dihydrospiro[azetidine-3,6'-pyrido[3,4-b]indol]-9'(5'H)-yl)benzamide

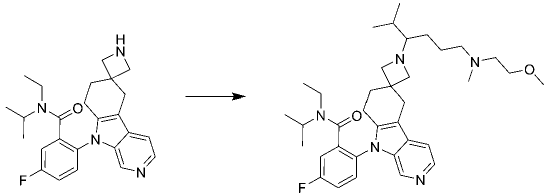

Intermediate 1 (80 mg), Intermediate 2 (84 mg) and a solution of acetic acid in ethanol (10%, 12 mg) were added to ethanol (1 mL), the mixture was stirred at room temperature for 1 hour. Sodium triacetoxyborohydride (170 mg) was added, and the mixture was stirred for 8 hours, concentrated under reduced pressure to remove the solvent. The residue was added to dichloromethane, the solution was washed sequentially with water and brine, dried over anhydrous sodium sulfate, concentrated under reduced pressure, and separated through silica gel column chromatography (dichloromethane/methanol, 15:1, containing 1% concentrated ammonia water) to obtain the target compound (36 mg). MS m/z [LC-MS]:606.42 [M+1].

### Example 2: N-ethyl-5-fluoro-N-isopropyl-2-(1-(6-((2-methoxyethyl)(methyl)amino)-2-m ethylhexan-3-yl)-6',9'-dihydrospiro[azetidine-3,8'-pyrimido[5,4-b]indol]-5'(7'H)-yl)benzamide

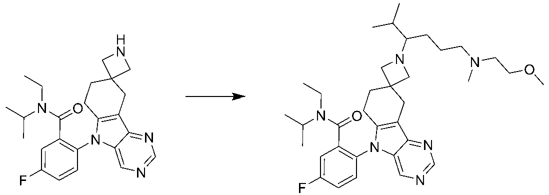

Following the method in Example 1, Intermediate 3 was used as the starting material to obtain the target compound. MS m/z [LC-MS]:607.41 [M+1]. ¹H NMR (400 MHz, CDCl₃), *δ* = 8.91-8.93 (m, 1H), 8.25-8.28 (m, 1H), 7.14-7.41 (m, 3H), 2.10-3.74 (m, 24H), 1.38-1.98 (m, 7H), 0.63-1.18 (m, 12H), 0.38-0.53 (m, 3H).

### Example 3: N-ethyl-5-fluoro-N-isopropyl-2-(1-(6-((2-methoxyethyl)(methyl)amino)-2-m ethylhexan-3-yl)-7',8'-dihydrospiro[azetidine-3,6'-[1,2,4]triazino[6,5-b]indole]-9'(5'H)-yl)benza mide

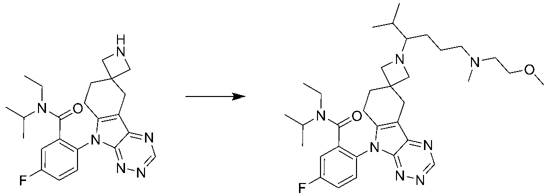

Following the method in Example 1, the target compound was obtained using Intermediate 4 as the starting material. MS m/z [LC-MS]:608.41 [M+1]. ¹H NMR (400 MHz, CDCl₃), *δ* = 9.37 (s, 0.7H), 9.36 (s, 0.3H), 7.38-7.42 (m, 1H), 7.23-7.29 (m, 1H), 7.14-7.19 (m, 1H), 3.84-3.98 (m, 1H), 3.42-3.53 (m, 2H), 2.93-3.36 (m, 10H), 2.72-2.84 (m, 1H), 2.49-2.64 (m, 3H), 1.97-2.40 (m, 7H), 1.68-1.82 (m, 1H), 1.42-1.58 (m, 2H), 1.15-1.38 (m, 4H), 1.04-1.09 (m, 3H), 0.78-0.92 (m, 7H), 0.52-065 (m, 5H).

Examples 4, 6-27, 33-34, 37-44, and 46-52 in the table below were prepared according to the synthetic method of Example 1, using the corresponding starting materials.

| **Example** | **Structure** | **MS [M+1] m/z** | **¹H NMR (400 MHz): *δ*** | **Starting Material** |
|---|---|---|---|---|
| Ex. **4** | 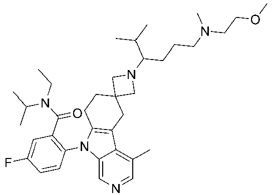 | 620.43 | (CDCl₃), 8.03 (s, 1H), 7.92-7.93 (m, 1H), 7.30-7.33 (m, 1H), 7.18-7.24 (m, 1H), 7.11-7.16 (m, 1H), 3.45-3.93 (m, 3H), 2.98-3.42 (m, 11H), 2.50-2.82 (m, 7H), 2.16-2.40 (m, 6H), 1.75-2.07 (m, 3H), 1.16-1.59 (m, 4H), 0.80-1.00 (m, 10H), 0.62-0.76 (m, 3H), 0.29-0.31 (m, 2H). | Intermediate **1** |
| | | | | Intermediate **5** |
| Ex. **6** | 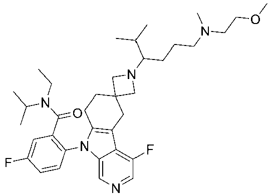 | 624.41 | (CDCl₃), 7.97-8.05 (m, 2H), 7.32-7.35 (m, 1H), 7.12-7.25 (m, 2H), 3.42-3.61 (m, 3H), 3.30 (s, 3H), 2.95-3.24 (m, 6H), 2.68-2.79 (m, 1H), 2.51 (t, J = 5.6 Hz, 2H), 2.26-2.37 (m, 3H), 2.22 (s, 3H), 1.88-2.07 (m, 3H), 1.65-1.77 (m, 1H), 1.40-1.53 (m, 2H), 1.10-1.36 (m, 3H), 0.78-1.00 (m, 11H), 0.66-0.74 (m, 3H), 0.29-0.31 (m, 2H). | Intermediate **1** |
| | | | | Intermediate **8** |
| Ex. **7** | 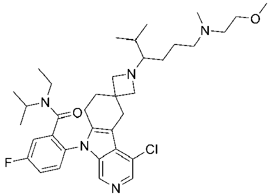 | 640.38 | (CDCl₃), 8.13 (s, 1H), 8.07 (s, 1H), 7.34 (dd, J = 8.8 Hz, 4.8 Hz, 1H), 7.20-7.28 (m, 1H), 7.16 (dd, J = 8.0 Hz, 2.8 Hz, 1H), 3.54-3.64 (m, 1H), 3.46-3.52 (m, 5H), 3.31-3.43 (m, 3.5H), 2.99-3.26 (m, 6H), 2.73-2.83 (m, 1H), 2.52-2.55 (m, 2H), 2.30-2.41 (m, 2.5H), 2.25 (s, 1.5H), 2.24 (s, 1.5H), 1.90-2.08 (m, 2H), 1.44-1.82 (m, 3H), 1.12-1.34 (m, 2H), 0.96-1.04 (m, 3H), 0.82-0.88 (m, 7H), 0.72-0.76 (m, 3H), 0.34-0.36 (m, 2H). | Intermediate **1** |
| | | | | Intermediate **9** |
| Ex. **8** | 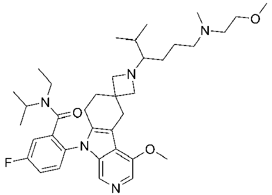 | 636.43 | (CD₃OD), 8.13 (s, 0.3H), 7.87 (s, 0.7H), 7.74 (s, 0.7H), 7.69 (s, 0.3H), 7.50 (dd, J = 8.4 Hz, 5.2 Hz, 0.7H), 7.35-7.46 (m, 1.3H), 7.31-7.33 (m, 1H), 3.97-4.00 (m, 3H), 3.53-3.70 (m, 1H), 3.49 (t, J = 5.6 Hz, 2H), 3.31-3.42 (m, 1H), 2.72-3.26 (m, 9H), 2.57 (t, J = 5.6 Hz, 2H), 2.32-2.43 (m, 3H), 2.25 (s, 3H), 2.15-2.22 (m, 1H), 1.78-2.08 (m, 3H), 1.48-1.60 (m, 2H), 1.16-1.41 (m, 3H), 0.99-1.04 (m, 3H), 0.78-0.95 (m, 7H), 0.67-0.74 (m, 3H), 0.36-0.39 (m, 3H). | Intermediate **1** |
| | | | | Intermediate **10** |
| Ex. **9** | 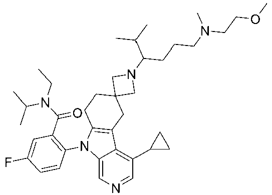 | 646.45 | | Intermediate **1** |
| | | | | Intermediate **11** |
| Ex. **10** | 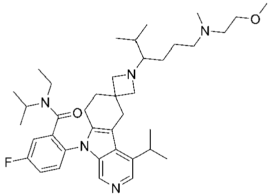 | 648.46 | (CDCl₃), 8.11 (s, 0.7H), 8.10 (s, 0.3H), 8.05 (s, 1H), 7.34-7.37 (m, 1H), 7.18-7.25 (m, 1H), 7.10-7.16 (m, 1H), 3.50-3.67 (m, 2H), 3.46 (t, J = 5.6 Hz, 2H), 3.00-3.42 (m, 10H), 2.65-2.76 (m, 1H), 2.53-2.58 (m, 2H), 2.29-2.40 (m, 3H), 2.26 (s, 3H), 2.12-2.22 (m, 1H), 1.90-2.08 (m, 2H), 1.74-1.85 (m, 1H), 1.46-1.58 (m, 2H), 1.26-1.42 (m, 8H), 0.92-0.96 (m, 3H), 0.79-0.89 (m, 7H), 0.59-0.67 (m, 3H), 0.20-0.35 (m, 3H). | Intermediate **1** |
| | | | | Intermediate **12** |
| Ex. **11** | 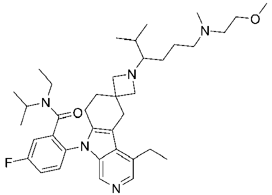 | 634.45 | | Intermediate **1** |
| | | | | Intermediate **13** |
| Ex. **12** | 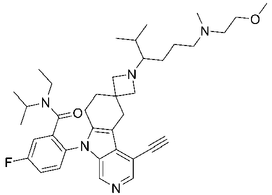 | 630.42 | | Intermediate **1** |
| | | | | Intermediate **14** |
| Ex. **13** | 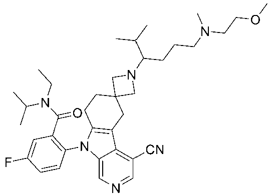 | 631.41 | | Intermediate **1** |
| | | | | Intermediate **15** |
| Ex. **14** | 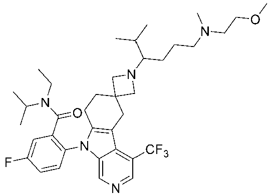 | 674.41 | | Intermediate **1** |
| | | | | Intermediate **16** |
| Ex. **15** | 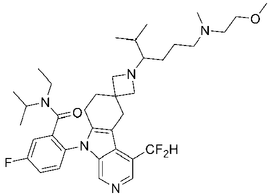 | 656.41 | (CD₃OD), 8.46-8.80 (brs, 1H), 7.82-8.04 (m, 1H), 7.53-7.65 (m, 1H), 7.38-7.50 (m, 2H), 6.82-7.12 (m, 1H), 4.02-4.38 (m, 4H), 3.55-3.82 (m, 3H), 3.02-3.54 (m, 10H), 2.75-2.98 (m, 6H), 2.46-2.73 (m, 1H), 2.09-2.42 (m, 3H), 1.79-1.95 (m, 2H), 1.50-1.78 (m, 2H), 1.04-1.14 (m, 6H), 0.96-1.03 (m, 3H), 0.72-0.92 (m, 3H), 0.61-0.71 (m, 1.5H), 0.48-0.60 (m, 1.5H). | Intermediate **1** |
| | | | | Intermediate **17** |
| Ex. **16** | 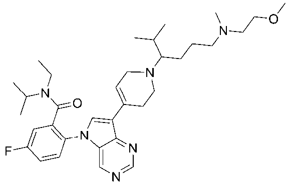 | 593.40 | (CDCl₃), 9.06 (s, 1H), 8.74 (s, 1H), 7.62 (s, 1H), 7.42-7.45 (m, 1H), 7.12-7.28 (m, 3H), 3.20-3.50 (m, 9H), 2.68-2.95 (m, 2H), 2.54 (t, J = 5.6 Hz, 2H), 2.44-2.50 (m, 2H), 2.36 (t, J = 7.2 Hz, 2H), 2.24 (s, 3H), 2.13-2.22 (m, 2H), 1.78-1.92 (m, 1H), 1.45-1.65 (m, 2H), 1.18-1.40 (m, 2H), 0.51-1.15 (m, 13H), 0.26 (d, J = 6.4 Hz, 2H). | Intermediate **1** |
| | | | | Intermediate **18** |
| Ex. **17** | 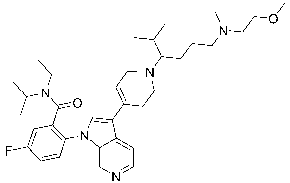 | 592.40 | | Intermediate **1** |
| | | | | Intermediate **19** |
| Ex. **18** | 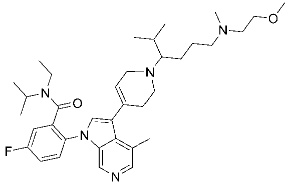 | 606.42 | (CDCl₃), 8.47 (s, 1H), 8.03-8.04 (m, 1H), 7.46-7.51 (m, 1H), 7.29-7.31 (m, 1H), 7.13-7.25 (m, 2H), 5.62-5.70 (m, 1H), 3.38-3.50 (m, 4H), 3.29-3.36 (m, 3H), 3.22-3.27 (m, 1.4H), 3.11-3.15 (m, 0.6H), 2.63-2.94 (m, 3H), 2.30-2.62 (m, 10H), 2.04-2.28 (m, 5H), 1.69-1.88 (m, 1H), 1.45-1.65 (m, 2H), 0.74-1.18 (m, 13H), 0.16 (d, J = 6.4 Hz, 2H). | Intermediate **1** |
| | | | | Intermediate **20** |
| Ex. **19** | 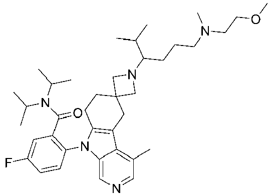 | 634.45 | (CDCl₃), 8.06 (s, 1H), 7.96 (s, 1H), 7.32-7.35 (m, 1H), 7.21-7.24 (m, 1H), 7.08 (dd, J = 8.0 Hz, 2.4 Hz, 1H), 3.75-3.94 (m, 3H), 3.64-3.74 (m, 2H), 3.51-3.62 (m, 1H), 3.40-3.45 (m, 1H), 3.35 (s, 3H), 3.10-3.28 (m, 2H), 2.84-3.02 (m, 3H), 2.67-2.82 (m, 5H), 2.52-2.65 (m, 4H), 2.36-2.48 (m, 2H), 1.60-2.22 (m, 7H), 1.37 (d, J = 7.2 Hz, 3H), 1.01-1.07 (m, 9H), 0.87-0.90 (m, 3H), 0.37 (d, J = 6.8 Hz, 3H). | Intermediate **1** |
| | | | | Intermediate **21** |
| Ex. **20** | 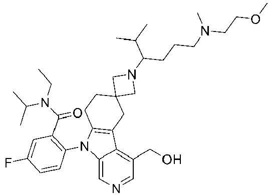 | 636.43 | (CDCl₃), 8.33 (s, 0.4 Hz, 1H), 8.28 (s, 0.6H), 7.61 (s, 0.4H), 7.13-7.31 (m, 3.6 H), 4.66-4.74 (m, 2H), 3.34-3.59 (m, 5H), 3.30-3.32 (m, 3H), 2.89-3.28 (m, 6H), 2.62-2.78 (m, 1H), 2.50-2.58 (m, 2H), 1.74-2.42 (m, 9H), 1.45-1.58 (m, 2H), 1.21-1.44 (m, 3H), 0.83-0.98 (m, 9H), 0.67-0.75 (m, 3.6H), 0.34 (t, J = 6.4 Hz, 1.2H), 0.26 (d, J = 6.4 Hz, 1.2H). | Intermediate **1** |
| | | | | Intermediate **22** |
| Ex. **21** | 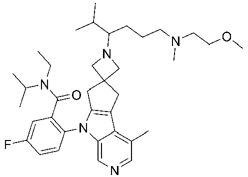 | 606.42 | (CDCl₃), 8.12-8.18 (m, 1H), 7.96-8.03 (m, 1H), 7.37-7.44 (m, 1H), 7.21-7.28 (m, 1H), 7.11-7.19 (m, 1H), 3.76-4.06 (m, 2H), 3.44-3.72 (m, 8H), 3.28-3.40 (m, 5H), 2.72-2.91 (m, 3H), 2.51-2.66 (m, 6H), 2.42-2.44 (m, 3H), 1.84-1.97 (m, 1H), 1.58-1.74 (m, 2H), 1.45-1.56 (m, 1H), 1.19-1.33 (m, 1H), 0.83-0.98 (m, 10H), 0.60-0.76 (m, 3H), 0.26 (t, J = 6.4 Hz, 2H). | Intermediate **1** |
| | | | | Intermediate **23** |
| Ex. **22** | 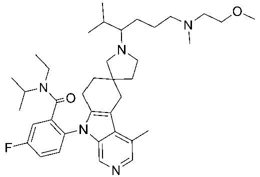 | 634.45 | (CDCl₃), 8.03-8.11 (m, 1H), 7.94 (s, 1H), 7.34-7.43 (m, 1H), 7.22-7.28 (m, 1H), 7.09-7.16 (m, 1H), 3.56-3.68 (m, 3H), 3.33-3.35 (m, 3H), 2.86-3.31 (m, 6H), 2.73-2.85 (m, 3H), 2.52-2.69 (m, 7H), 2.35-2.50 (m, 5H), 1.54-2.17 (m, 9H), 0.96-1.06 (m, 9H), 0.63-0.73 (m, 3H), 0.38-0.50 (m, 3H). | Intermediate **1** |
| | | | | Intermediate **24** |
| Ex. **23** | 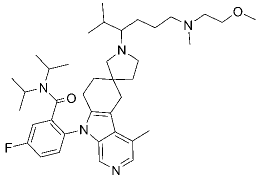 | 648.47 | (CDCl₃), 8.02 (s, 0.5H), 8.01 (s, 0.5H), 7.92 (s, 1H), 7.30-7.35 (m, 1H), 7.17-7.22 (m, 1H), 7.06-7.10 (m, 1H), 3.45-3.64 (m, 3H), 3.32-3.34 (m, 3H), 2.10-3.28 (m, 20H), 1.35-2.04 (m, 12H), 0.80-1.04 (m, 12H), 0.28-0.40 (m, 3H). | Intermediate **1** |
| | | | | Intermediate **25** |
| Ex. **24** | 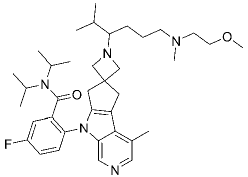 | 620.43 | (CD₃OD), 8.66-8.69 (m, 1H), 8.08-8.11 (m, 1H), 7.62-7.74 (m, 1H), 7.37-7.56 (m, 2H), 3.68-3.74 (m, 2H), 2.86-3.66 (m, 21H), 2.72 (s, 3H), 1.54-1.94 (m, 5H), 1.36-1.41 (m, 3H), 0.98-1.17 (m, 9H), 0.66-0.86 (m, 3H), 0.42-0.58 (m, 3H). | Intermediate **1** |
| | | | | Intermediate **26** |
| Ex. **25** | 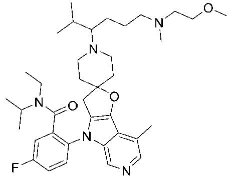 | 536.43 | (CD₃OD), 8.71 (s, 0.5H), 8.60 (s, 0.5H), 8.01 (s, 0.5H), 8.00 (s, 0.5H), 7.66-7.76 (m, 1H), 7.42-7.51 (m, 2H), 2.97-3.80 (m, 18H), 2.91 (s, 3H), 2.73 (s, 3H), 2.11-2.64 (m, 5H), 1.50-2.09 (m, 4H), 1.26-1.44 (m, 2H), 1.02-1.18 (m, 7H), 0.75-0.95 (m, 4H), 0.44-0.56 (m, 2H). | Intermediate **1** |
| | | | | Intermediate **27** |
| Ex. **26** | 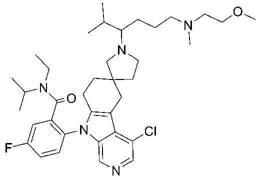 | 554.39 | (CD₃OD), 8.66 (s, 1H), 8.35 (s, 1H), 7.64-7.76 (m, 1H), 7.38-7.54 (m, 2H), 3.58-4.08 (m, 5H), 3.03-3.56 (m, 14H), 2.52-3.00 (m, 5H), 1.52-2.41 (m, 9H), 0.44-1.24 (m, 15H). | Intermediate **1** |
| | | | | Intermediate **28** |
| Ex. **27** | 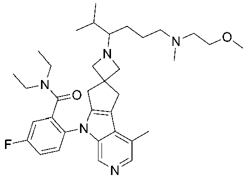 | 592.40 | (CD₃OD), 8.58 (s, 0.5H), 8.41 (s, 0.5H), 8.08 (s, 0.5H), 8.07 (s, 0.5H), 7.65-7.74 (m, 1H), 7.42-7.54 (m, 2H), 4.38-4.56 (m, 4H), 2.85-3.76 (m, 21H), 2.70 (s, 1.5H), 2.69 (s, 1.5H), 1.96-2.22 (m, 1H), 1.81-1.95 (m, 2H), 1.54-1.78 (m, 2H), 0.96-1.10 (m, 8.5H), 0.82-0.94 (m, 1H), 0.72 (d, J = 6.4 Hz, 1H), 0.42-0.50 (m, 1.5H). | Intermediate **1** |
| | | | | Intermediate **29** |
| Ex. **33** | 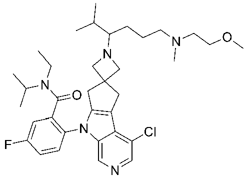 | 626.36 | (CDCl₃), 8.10-8.15 (m, 2H), 7.28-7.42 (m, 1H), 7.10-7.24 (m, 2H), 3.18-3.63 (m, 13H), 3.04-3.15 (m, 1H), 2.71-2.85 (m, 2H), 2.51 (t, J = 5.6 Hz, 2H), 2.28-2.32 (m, 2H), 2.22 (s, 3H), 1.90-1.98 (m, 1H), 1.54-1.75 (m, 1H), 1.40-1.51 (m, 2H), 1.11-1.32 (m, 2H), 0.70-0.99 (m, 13H), 0.23-0.27 (m, 2H). | Intermediate **1** |
| | | | | Intermediate **30** |
| Ex. **34** | 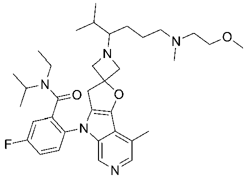 | 608.40 | (CDCl₃), 8.16 (s, 1H), 7.96 (s, 1H), 7.34-7.45 (m, 1H), 7.10-7.24 (m, 2H), 3.71-3.80 (m, 1H), 3.36-3.55 (m, 6H), 3.33 (s, 1.5H), 3.32 (s, 1.5H), 3.19-3.28 (m, 1H), 2.80-2.91 (m, 1H), 2.51-2.56 (m, 4H), 2.16-2.38 (m, 8H), 2.02-2.09 (m, 1H), 1.66-1.81 (m, 1H), 1.42-1.56 (m, 2H), 1.15-1.36 (m, 2H), 0.81-0.93 (m, 13H), 0.17-0.20 (m, 2H). | Intermediate **1** |
| | | | | Intermediate **31** |
| Ex. **37** | 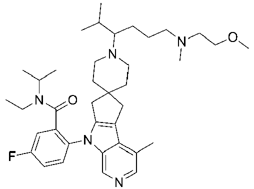 | 634.45 | (CDCl₃), 8.14 (s, 1H), 7.99 (s, 1H), 7.32-7.41 (m, 1H), 7.12-7.26 (m, 2H), 3.51-3.58 (m, 3H), 3.31-3.40 (m, 4H), 3.05 (d, J = 17.2 Hz, 1H), 2.80-2.88 (m, 3H), 2.64-2.78 (m, 5H), 2.50-2.63 (m, 6H), 2.40-2.47 (m, 4H), 2.17-2.26 (m, 1H), 1.44-1.94 (m, 7H), 1.17-1.40 (m, 2H), 0.82-0.96 (m, 13H), 0.23 (d, J = 6.4 Hz, 2H). | Intermediate **1** |
| | | | | Intermediate **32** |
| Ex. **38** | 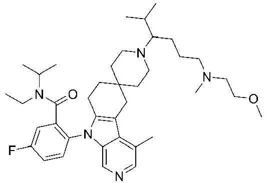 | 648.46 | (CDCl₃), 8.04 (s, 1H), 7.92-7.93 (m, 1H), 7.31-7.35 (m, 1H), 7.20-7.25 (m, 1H), 7.12-7.17 (m, 1H), 3.49-3.66 (m, 3H), 3.30-3.38 (m, 4H), 2.16-3.03 (m, 20H), 1.46-1.96 (m, 9H), 1.16-1.41 (m, 2H), 0.62-1.00 (m, 13H), 0.37 (d, J = 6.8 Hz, 2H). | Intermediate **1** |
| | | | | Intermediate **33** |
| Ex. **39** | 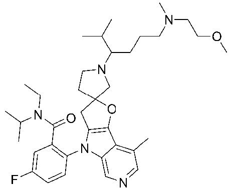 | 622.41 | | Intermediate **1** |
| | | | | Intermediate **34** |
| Ex. **40** | 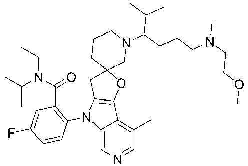 | 636.43 | (CDCl₃), 8.14 (s, 1H), 7.94 (s, 1H), 7.38-7.46 (m, 1H), 7.11-7.25 (m, 2H), 3.28-3.60 (m, 6H), 2.14-3.08 (m, 19H), 1.18-2.12 (m, 9H), 1.78-1.12 (m, 13H), 0.19-0.25 (m, 2H). | Intermediate **1** |
| | | | | Intermediate **35** |
| Ex. **41** | 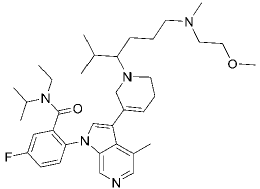 | 606.42 | (CDCl₃), 8.49 (s, 1H), 8.05 (s, 1H), 7.47-7.52 (m, 1H), 7.35 (d, J = 1.6 Hz, 1H), 7.23-7.28 (m, 1H), 7.15-7.19 (m, 1H), 5.69-5.74 (m, 1H), 3.58-3.81 (m, 2H), 3.19-3.54 (m, 6H), 3.02-3.15 (m, 2H), 2.50-3.01 (m, 15H), 1.46-2.33 (m, 5H), 0.76-0.98 (m, 13H), 0.19 (d, J = 6.4 Hz, 2H). | Intermediate **1** |
| | | | | Intermediate **36** |
| Ex. **42** | 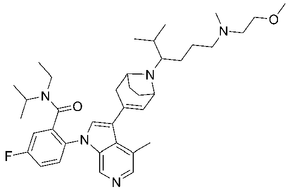 | 632.43 | | Intermediate **1** |
| | | | | Intermediate **37** |
| Ex. **43** | 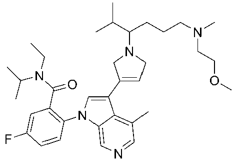 | 592.40 | | Intermediate **1** |
| | | | | Intermediate **38** |
| Ex. **44** | 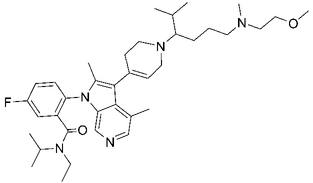 | 620.43 | | Intermediate **1** |
| | | | | Intermediate **39** |
| Ex. **46** | 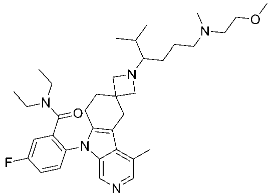 | 606.42 | (CD₃OD), 8.02 (s, 1H), 7.82 (s, 1H), 7.47-7.55 (m, 1H), 7.38-7.46 (m, 1H), 7.31-7.37 (m, 1H), 3.49 (t, J = 5.6 Hz,3H), 3.18-3.28 (m, 7H), 2.70-2.87 (m, 3H), 2.65 (s, 3H), 2.58 (t, J = 5.6 Hz,2H), 2.39 (t, J = 8.0 Hz,2H), 2.26 (s, 3H), 2.18-2.26 (m, 1H), 1.94-2.08(m, 2H),1.80-1.90 (m, 1H), 1.47-1.58 (m, 2H), 1.20-1.42 (m, 4H), 1.18(s, 3H), 0.83-0.98 (m, 9H), 0.33-0.39 (m, 2H). | Intermediate **1** |
| | | | | Intermediate **40** |
| Ex.**47** | 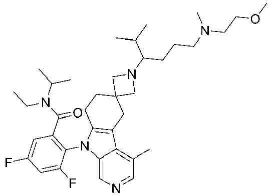 | 638.42 | (CDCl₃), 8.01 (s, 1H), 7.96 (s, 1H), 7.00-7.10 (m, 1H), 6.90-7.10 (m, 1H), 3.82-3.90 (m, 0.2H), 3.56-3.64 (m, 0.8H), 3.40-3.48 (m, 2.8H), 3.28-3.40 (m, 3.8H), 2.95-3.25 (m, 6.4H), 2.72-2.82 (m, 1H), 2.58-2.66 (m, 3H), 2.52-2.58 (m, 2H), 2.28-2.38 (m, 3H), 2.25 (s, 3H), 1.85-2.10 (m, 3H), 1.70-1.82 (m, 1H), 1.42-1.56 (m, 2H), 1.12-1.36 (m, 3H), 0.65-1.04 (m, 12H), 0.28 (m, 2H). | Intermediate **1** |
| | | | | Intermediate **41** |
| Ex.**48** | 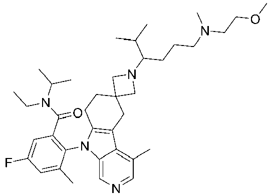 | 634.45 | . | Intermediate **1** |
| | | | | Intermediate **42** |
| Ex.**49** | 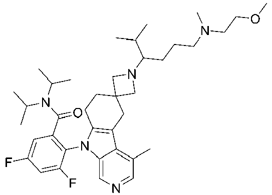 | 652.44 | | Intermediate **1** |
| | | | | Intermediate **43** |
| Ex.**50** | 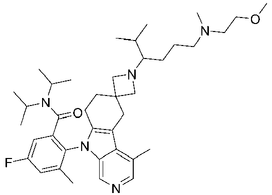 | 648.46 | | Intermediate **1** |
| | | | | Intermediate **44** |
| Ex.**51** | 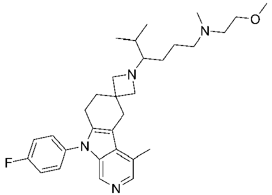 | 507.35 | (CDCl₃), 8.30 (s, 1H), 7.98 (s, 1H), 7.26-7.33 (m, 2H), 7.18-7.25 (m, 2H), 3.40-3.70 (m, 5H), 3.25-3.37 (m, 4H), 1.86-2.74 (m, 16H), 1.22-1.46 (m, 6H), 0.88-1.02 (m, 6H). | Intermediate **1** |
| | | | | Intermediate **45** |
| Ex.**52** | 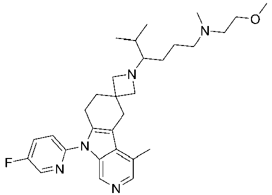 | 508.34 | | Intermediate **1** |
| | | | | Intermediate **46** |

### Example 5: (R)-N-ethyl-5-fluoro-N-isopropyl-2-(1-(6-((2-methoxyethyl)(methyl)amino)-2-methylhexan-3-yl)-7',8'-dihydrospiro[azetidine-3,6'-pyrido[3,4-b]indol]-9'(5'H)-yl)benzamide

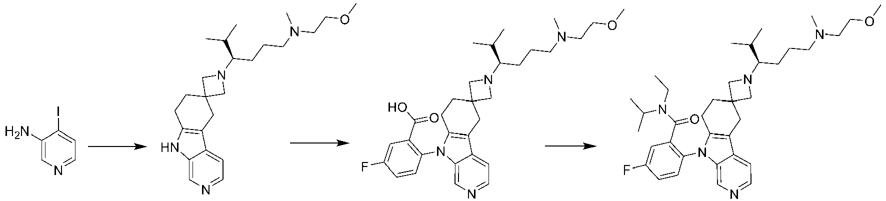

### Step 1: (R)-N-(2-methoxyethyl)-N,5-dimethyl-4-(5',7',8',9'-tetrahydrospiro[azetidine-3,6'-pyrido[3,4-b]indol]-1-yl)hexan-1-amine

Following the method of Step 1 in Intermediate 2, Intermediate 7 was used as the starting material to obtain the target product. MS m/z [LC-MS]: 399.31 [M+1].

### Step 2: (R)-5-fluoro-2-(1-(6-((2-methoxyethyl)(methyl)amino)-2-methylhexan-3-yl)-7',8' -dihydrospiro[azetidine-3,6'-pyrido[3,4-b]indol]-9'(5'H)-yl)benzoic acid

Following the method of Step 2 in Intermediate 2, (R)-N-(2-methoxyethyl)-N,5-dimethyl-4-(5',7',8',9'-tetrahydrospiro[azetidine-3,6'-pyrido[3,4-b]indol]-1-yl)hexan-1-amine was used as the starting material to obtain the target product. MS m/z [LC-MS]: 537.32 [M+1].

### Step 3: (R)-N-ethyl-5-fluoro-N-isopropyl-2-(1-(6-((2-methoxyethyl)(methyl)amino)-2-me thylhexan-3-yl)-7',8'-dihydrospiro[azetidine-3,6'-pyrido[3,4-b]indol]-9'(5'H)-yl)benzamide

Following the method of step 3 in Intermediate 2, (R)-5-fluoro-2-(1-(6-((2-methoxyethyl) (methyl)amino)-2-methylhexan-3-yl)-7',8'-dihydrospiro[azetidine-3,6'-pyrido[3,4-b]indol]-9'(5'*H*)-yl)benzoic acid was used as the starting material to obtain the target compound. MS m/z [LC-MS]: 606.42 [M+1].

### Example 28: (S)-N-ethyl-5-fluoro-N-isopropyl-2-(1-(6-((2-methoxyethyl)(methyl)amino) -2-methylhexan-3-yl)-4'-methyl-7',8'-dihydrospiro[azetidine-3,6'-pyrido[3,4-b]indol]-9'(5'H)-yl) benzamide

### Example 29: (R)-N-ethyl-5-fluoro-N-isopropyl-2-(1-(6-((2-methoxyethyl)(methyl)amino) -2-methylhexan-3-yl)-4'-methyl-7',8'-dihydrospiro[azetidine-3,6'-pyrido[3,4-b]indol]1-9'(5'H)-yl) benzamide

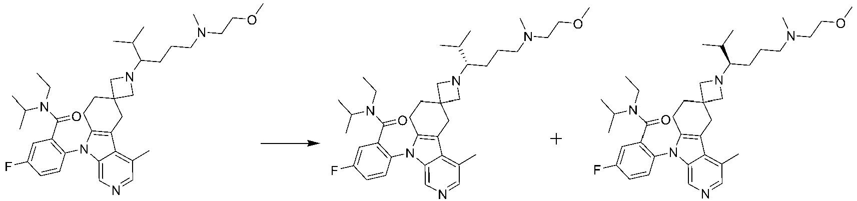

Example 4 was separated using an IC-5 chiral preparative column (5 µm, 10 x 250 mm), with a mobile phase of n-hexane/ethanol/diethylamine = 75:25:0.1 at a flow rate of 4.7 mL/min, to obtain (S)-N-ethyl-5-fluoro-N-isopropyl-2-(1-(6-((2-methoxyethyl)(methyl)amino)-2-methyl hexan-3-yl)-4'-methyl-7',8'-dihydrospiro[azetidine-3,6'-pyrido[3,4-*b*]indol]-9'(5'*H*)-yl)benzamide. T_{R} = 14.8 minutes. MS m/z [LC-MS]: 620.43 [M+1]. ¹H NMR (400 MHz, CD₃OD), *δ* = 8.26 (s, 0.2H), 8.00 (s, 0.8H), 7.80-7.85 (m, 1H), 7.48-7.51 (m, 0.8H), 7.37-7.43 (m, 1.2H), 7.30-7.35 (m, 1H), 3.54-3.82 (m, 1H), 3.49 (t, *J=* 5.6 Hz, 2H), 3.12-3.41 (m, 9H), 2.93-3.09 (m, 1H), 2.73-2.90 (m, 1H), 2.66 (s, 2.4H), 2.65 (s, 0.6H), 2.58 (t, *J* = 5.6 Hz, 2H), 2.29-2.46 (m, 3H), 2.26 (s, 3H), 2.15-2.24 (m, 1H), 1.80-2.08 (m, 3H), 1.48-1.61 (m, 2H), 1.15-1.43 (m, 3H), 0.98-1.05 (m, 3H), 0.93 (d, *J* = 6.8 Hz, 3H), 0.74-0.91 (m, 5H), 0.67 (t, *J=* 7.2 Hz, 2H), 0.34-0.38 (m, 2H).

(R)-N-ethyl-5-fluoro-N-isopropyl-2-(1-(6-((2-methoxyethyl)(methyl)amino)-2-methylhexan-3-yl)-4'-methyl-7',8'-dihydrospiro[azetidine-3,6'-pyrido[3,4-b]indol]-9'(5'*H*)-yl)benzamide was also obtained. T_{R} = 23.2 minutes. MS m/z [LC-MS]: 620.43 [M+1]. ¹H NMR (400 MHz, CD₃OD), *δ* = 8.26 (s, 0.2H), 8.00 (s, 0.8H), 7.80-7.85 (m, 1H), 7.48-7.51 (m, 0.8H), 7.37-7.43 (m, 1.2H), 7.30-7.35 (m, 1H), 3.54-3.82 (m, 1H), 3.49 (t, *J* = 5.6 Hz, 2H), 3.12-3.41 (m, 9H),2.93-3.09 (m, 1H), 2.73-2.90 (m, 1H), 2.66 (s, 2.4H), 2.65 (s, 0.6H), 2.58 (t, *J* = 5.6 Hz, 2H), 2.29-2.46 (m, 3H), 2.26 (s, 3H), 2.15-2.24 (m, 1H), 1.80-2.08 (m, 3H), 1.48-1.61 (m, 2H), 1.15-1.43 (m, 3H), 0.98-1.05 (m, 3H), 0.93 (d, *J* = 6.8 Hz, 3H), 0.74-0.91 (m, 5H), 0.67 (t, *J =* 7.2 Hz, 2H), 0.34-0.38 (m, 2H).

### Example 30: (S)-2-(4'-chloro-1-(6-((2-methoxyethyl)(methyl)amino)-2-methylhexan-3-y l)-7',8'-dihydrospiro[azetidine-3,6'-pyrido[3,4-b]indol]-9'(5'H)-yl)-N-ethyl-5-fluoro-N-isopropyl benzamide

### Example 31: (R)-2-(4'-chloro-1-(6-((2-methoxyethyl)(methyl)amino)-2-methylhexan-3-y l)-7',8'-dihydrospiro[azetidine-3,6'-pyrido[3,4-b]indol]-9'(5'H)-yl)-N-ethyl-5-fluoro-N-isopropyl benzamide

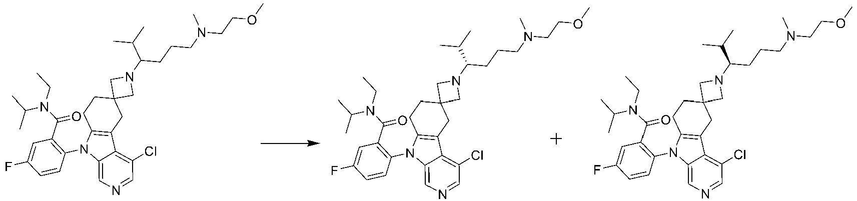

Example 7 was separated using an IC-5 chiral preparative column (5 µm, 10 x 250 mm), with a mobile phase of n-hexane/ethanol/diethylamine = 75:25:0.1 at a flow rate of 4.7 mL/min, to obtain *(S)*-2-(4'-chloro-1-(6-((2-methoxyethyl)(methyl)amino)-2-methylhexan-3-yl)-7',8'-dihydrospiro[azetidine-3,6'-pyrido[3,4-b]indol]-9'(5'H)-yl)-N-ethyl-5-fluoro-N-isopropylbenzamide. T_{R} = 15.5 minutes. MS m/z [LC-MS]: 640.38 [M+1]. ¹H NMR (400 MHz, CDCl₃), *δ* = 8.46 (s, 0.2H), 8.13 (s, 0.8H), 8.12 (s, 0.2H), 8.06 (s, 0.8H), 7.32-7.35 (m, 0.8H), 7.14-7.28 (m, 2.2H), 3.86-3.96 (m, 0.2H), 3.53-3.66 (m, 0.8H), 3.30-3.51 (m, 6H), 2.98-3.28 (m, 6H), 2.72-2.84 (m, 1H), 2.54 (t, *J* = 5.6 Hz, 2H), 2.28-2.42 (m, 3H), 2.24 (s, 3H), 1.68-2.10 (m, 4H), 1.43-1.56 (m, 2H), 1.12-1.36 (m, 3H), 0.94-1.06 (m, 3.2H), 0.81-0.92 (m, 6.2H), 0.66-0.78 (m, 3H), 0.31-0.50 (m, 2.6H).

*(R)*-2-(4'-chloro-1-(6-((2-methoxyethyl)(methyl)amino)-2-methylhexan-3-yl)-7',8'-dihydrospiro[azetidine-3,6'-pyrido[3,4-b]indol]-9'(5'H)-yl)-N-ethyl-5-fluoro-N-isopropylbenzamide was also obtained. T_{R} = 24.1 minutes. MS m/z [LC-MS]: 640.38 [M+1]. ¹H NMR (400 MHz, CD₃OD), *δ* = 8.44 (s, 0.3H), 8.28 (s, 0.7H), 8.13 (s, 1H), 7.76-7.81 (m, 4H), 7.51-7.64 (m, 1H), 7.33-7.50 (m, 8H), 4.01-4.44 (m, 4H), 3.61-3.89 (m, 3H), 2.88-3.60 (m, 13H), 2.89 (s, 3H), 2.48-2.76 (m, 1H), 2.08-2.44 (m, 3H), 1.80-2.06 (m, 2H), 1.54-1.78 (m, 2H), 1.06-1.13 (m, 6.6H), 0.99 (d, J = 6.8 Hz, 3H), 0.74-0.86 (m, 1.6H), 0.52-0.70 (m, 3H), 0.34-0.48 (m, 0.8H).

### Example 32: N-ethyl-5-fluoro-N-isopropyl-2-(3-(1-(6-((2-methoxyethyl)(methyl)amino)-2-methylhexan-3-yl)piperidin-4-yl)-1H-pyrrolo[2,3-c]pyridin-1-yl)benzamide

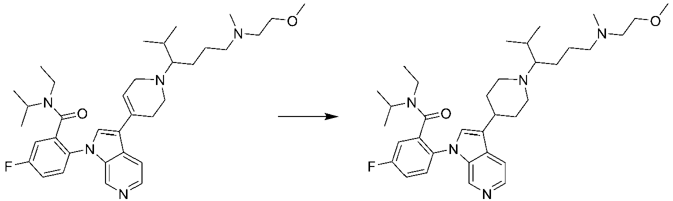

Example 18 (30 mg), palladium on carbon (10%, 6 mg) were added to methanol (3 mL). The air in the reactor was replaced with hydrogen, and the mixture was stirred at room temperature under 1 atm of hydrogen for 8 hours. The palladium on carbon was removed by filtration through celite. The filtrate was concentrated under reduced pressure and then separated by silica gel thin-layer chromatography (dichloromethane/methanol, 15:1, containing 1% concentrated ammonia water) to obtain the target compound (24 mg). MS m/z [LC-MS]:594.42 [M+1]. ¹H NMR (400 MHz, CDCl₃), *δ* = 8.49 (s, 1H), 8.03 (s, 1H), 7.44-7.47 (m, 1H), 7.40 (s, 1H), 7.22-7.26 (m, 1H), 7.14-7.17 (m, 1H), 3.78-3.86 (m, 3H), 3.58-3.69 (m, 2H), 3.43-3.56 (m, 1H), 3.30-3.42 (m, 5H), 2.72-3.24 (m, 10H), 2.68 (s, 3H), 2.25-2.38 (m, 2H), 1.45-2.10 (m, 7H), 0.76-1.38 (m, 15H).

### Example 35: (S)-N-ethyl-5-fluoro-N-isopropyl-2-(1-(6-((2-methoxyethyl)(methyl)amino) -2-methylhexan-3-yl)-4'-methyl-5',7'-dihydro-8'H-spiro[azetidine-3,6'-cyclopenta[4,5]pyrrolo[2, 3-c]pyridin]-8'-yl)benzamide

### Example 36: (R)-N-ethyl-5-fluoro-N-isopropyl-2-(1-(6-((2-methoxyethyl)(methyl)amino) -2-methylhexan-3-yl)-4'-methyl-5',7'-dihydro-8'H-spiro[azetidine-3,6'-cyclopenta[4,5]pyrrolo[2, 3-c]pyridin]-8'-yl)benzamide

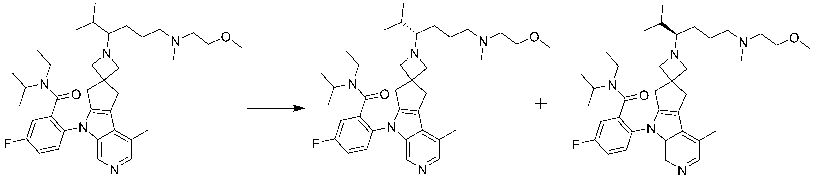

Example 21 was separated using an IC-5 chiral preparative column (5 µm, 10 x 250 mm), with a mobile phase of n-hexane/ethanol/diethylamine = 75:25:0.1 at a flow rate of 4.7 mL/min, to obtain (S)-N-ethyl-5-fluoro-N-isopropyl-2-(1-(6-((2-methoxyethyl)(methyl)amino)-2-methylhexan-3-yl)-4'-methyl-5',7'-dihydro-8'*H*-spiro[azetidine-3,6'-cyclopenta[4,5]pyrrolo[2,3-c]pyridin]-8'-yl)benzamide. T_{R} = 14.3 minutes. MS m/z [LC-MS]: 606.42 [M+1]. (R)-N-ethyl-5-fluoro-*N*-isopropyl-2-(1-(6-((2-methoxyethyl)(methyl)amino)-2-methylhexan-3-yl)-4'-methyl-5',7'-dihydro-8'*H*-spiro[azetidine-3,6'-cyclopenta[4,5]pyrrolo[2,3-c]pyridin]-8'-yl)benzamide was also obtained. T_{R} = 22.5 minutes. MS m/z [LC-MS]: 606.42 [M+1].

### Example 45: N-ethyl-5-fluoro-N-isopropyl-2-(7-(1-(6-((2-methoxyethyl)(methyl)amino)-2-methylhexan-3-yl)piperidin-4-yl)-5H-pyrrolo[3,2-d]pyrimidin-5-yl)benzamide

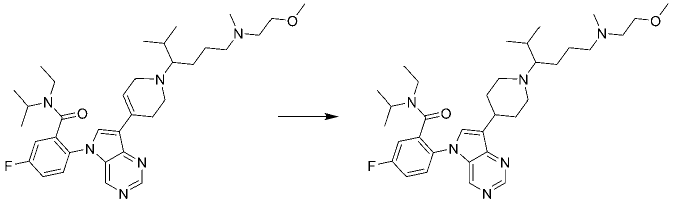

Following the method of Example 32, Example 16 was used as the starting material to obtain the target compound. MS m/z [LC-MS]:595.41 [M+1]. ¹H NMR (400 MHz, CDCl₃), *δ* = 9.01 (s, 1H), 8.73 (s, 0.8H), 8.71 (s, 0.2H), 7.50 (s, 0.2H), 7.49 (s, 0.8H), 7.40-7.45 (m, 1H), 7.17-7.28 (m, 2H), 4.20-4.30 (m, 0.2H), 3.39-3.53 (m, 2.8H), 3.35 (s, 2.4H), 3.33 (s, 0.6H), 2.78-3.02 (m, 3H), 2.54-2.67 (m, 3.8H), 2.37-2.44 (m, 2.2H), 2.29 (s, 2.4H), 2.28 (s, 0.6H), 2.02-2.14 (m, 3H), 1.40-1.85 (m, 6H), 1.18-1.38 (m, 2H), 1.02-1.14 (m, 1H), 0.78-0.96 (m, 13H), 0.28 (d, *J=* 6.8 Hz, 2H).

### Biological Testing

### Biochemical Assay for Inhibition of Wild-Type Menin-MLL Binding by Compounds

Wild-type Menin binds with high affinity to the conserved N-terminal sequence of MLL. Compounds bind to Menin by competing with the MLL N-terminus. FITC-MLL (4-43) (synthesized by GenScript) contains the conserved sequence responsible for MLL binding to Menin. We established a method to detect the binding activity between Menin and MLL using a fluorescence polarization binding assay (FP assay), which was then used to evaluate the inhibitory activity of compounds. The specific procedure is as follows:
Compounds were serially diluted 5-fold in 100% DMSO starting from 1 mM (8 concentrations in total). For each concentration, 2 µL was added to 48 µL of reaction buffer (50 mM NaCl, 50 mM Tris (pH 7.5), 0.05% Tween-20, 1 mM DTT) and mixed thoroughly to obtain 4× compound solutions (reaching final assay concentrations of 10000, 2000, 400, 80, 16, 3.2, 0.64, 0 nM) for later use. Use reaction buffer to prepare 2× Menin to a final assay concentration of 5 nM, and 4× FITC-MLL (4-43) to a final assay concentration of 2 nM. Add 5 µL of the 4× compound solution to a 384-well plate (OptiPlate-384, purchased from PerkinElmer). Then add 10 µL of the 2× Menin solution and centrifuge. After that, add 5 µL of FITC-MLL (4-43) and centrifuge again to initiate the reaction. Incubate at 23 °C for 1 hour protected from light. After the reaction, the signal was read on an EnVision 2104 Multilabel Reader (purchased from PerkinElmer) at an excitation wavelength of 480 nm and an emission wavelength of 535 nm. The data were analyzed using GraphPad Prism (data analysis software) to obtain the IC₅₀ values of the compounds.

### Biochemical Assay for Inhibition of Mutant Menin-MLL Binding by Compounds

The Menin^{mut} DNA sequence was cloned into the pET28a vector via restriction enzyme digestion and ligation. The recombinant protein was expressed using the BL21 E. coli system and purified by Ni-NTA to obtain Menin^{mut}. Menin binds with high affinity to the conserved N-terminal sequence of MLL, and compounds bind to Menin by competing with the MLL N-terminus. FITC-MLL (4-43) (synthesized by GenScript) contains the conserved sequence responsible for MLL binding to Menin. We employed a fluorescence polarization binding assay (FP assay) based on the principle that the binding of FITC-MLL (4-43) to Menin^{mut} increases the molecular weight and enhances fluorescence, to evaluate the inhibitory effect of compounds on the binding between Menin^{mut} and MLL. The detailed procedure is as follows:
Compounds were dissolved in DMSO to a final concentration of 10 mM. The 10 mM compound solution was then diluted 10-fold with DMSO, followed by 5-fold serial dilutions to obtain a total of 8 concentrations. For each concentration, 2 µL was added to 48 µL of reaction buffer (50 mM NaCl, 50 mM Tris, pH 7.5, 0.05% Tween-20, 1 mM DTT) and mixed thoroughly to obtain 4× compound solutions (reaching final assay concentrations of 10000, 2000, 400, 80, 16, 3.2, 0.64, 0 nM) for later use. Use reaction buffer to prepare 2× Menin^{mut} to a final assay concentration of 5 nM, and 4× FITC-MLL (4-43) to a final assay concentration of 2 nM. Add 5 µL of the 4× compound solution to a 384-well plate (OptiPlate-384, purchased from PerkinElmer). Then add 10 µL of the 2× Meninmut solution and centrifuge. After that, add 5 µL of FITC-MLL (4-43) and briefly centrifuge to initiate the reaction. Incubate at 23 °C for 1 hour protected from light. After the reaction, the signal was read on an EnVision 2104 Multilabel Reader (purchased from PerkinElmer) at an excitation wavelength of 480 nm and an emission wavelength of 535 nm. For each compound, the inhibitory effect was determined at 8 concentrations. The data were processed using GraphPad Prism software to calculate the half-maximal inhibitory concentration (IC50) of the compound for Menin-MLL binding.

Note: x denotes multiplication, indicating a multiple.

**Table 1. Inhibitory activity of the example compounds on wild-type and mutant Menin-MLL protein binding.**

| **Example** | **IC₅₀ (nM)** | |
|---|---|---|
| | Menin^{wt} | Menin^{M327I} |
| **4** | 8.0 | 3.8 |
| **6** | 10.0 | 7.4 |
| **7** | 6.2 | 3.1 |
| **19** | 8.5 | 2.8 |
| **21** | 11.1 | 9.0 |
| **22** | 11.2 | 3.3 |
| **23** | 10.9 | 3.7 |
| **24** | 28 | 11.4 |
| **25** | 5.4 | 4.7 |
| **26** | 1.8 | 4.3 |
| **29** | 3.4 | 1.9 |
| **30** | 10.7 | 19.9 |
| **31** | 1.9 | 2.5 |
| **32** | 10.4 | 6.0 |
| **33** | 8.1 | 6.1 |
| **35** | 8.1 | 4.5 |
| **36** | 4.0 | 2.9 |
| **37** | 5.6 | 4.6 |
| **38** | 8.1 | 6.8 |

Table 1 lists the IC₅₀ values of the inhibitory activity against wild-type and mutant Menin-MLL protein binding for the example compounds of the present invention. The data indicate that the compounds provided by the present invention exhibit strong inhibitory activity against wild-type and mutant Menin-MLL protein binding.

### Determination of the inhibitory activity of the compounds on cell proliferation of MV-4-11 cells expressing wild-type Menin protein:

MV-4-11 cells are human acute myeloid leukemia cells that contain the MLL-AF4 fusion protein. In this experiment, after incubating different concentrations of the compounds with MV-4-11 cells, the CellTiter-Blue^{®} detection reagent from Promega was used to establish a screening method for the inhibition of MV-4-11 cell proliferation.

MV-4-11 cells were cultured in RPMI-1640 medium (Biological Industries, Cat. No. 01-100-1ACS) supplemented with 10% fetal bovine serum (Biological Industries, Cat. No. 04-001-1ACS), under conditions of 37°C and 5% CO₂. The cells were subcultured 2 - 3 times per week. Cells were seeded into 96-well cell culture plates (Corning^{®}) at 5,000 cells per well in 195 µL/well and incubated at 37°C with 5% CO₂. After 24 hours, compounds were serially diluted 3-fold in 100% DMSO starting from 10 mM (10 concentrations in total). Then, 4 µL of each compound concentration was added to 96 µL of RPMI-1640 medium and mixed well. Subsequently, 5 µL of the diluted compound from each concentration was added to the prepared cell suspension, and the compounds were co-incubated with the cells in a cell culture incubator for 72 hours (3 days). Then, 35 µL of CellTiter-Blue reagent (Promega, Cat. No. G8082) was added, and the reaction was incubated at 37°C with 5% CO₂ for 4 hours. Fluorescence values were read on a BMG Clariostar Microplate Reader, and the data were processed using GraphPad Prism software to calculate the IC₅₀ value of the compound for cell proliferation inhibition.

Note: * refers to multiplication, indicating a multiple.

### Determination of the inhibitory activity of the compounds on cell proliferation of MOLM13 cells expressing mutant Menin protein:

The Menin^{M327I} DNA sequence was inserted into the lentiviral vector pCDHL-CMV-3xFlag-puroR via restriction enzyme digestion and ligation. The construct was co-transfected with psPAX2/pMD2G into 293T cells. After 48 hours, the virus was harvested and used to infect MOLM13 cells. Pool and monoclonal selections were performed using puromycin to ultimately obtain a stable cell line stably expressing the Menin^{M327I} recombinant protein.

MOLM13 cells are human acute myeloid leukemia cells that contain the MLL-AF9 fusion protein. In this experiment, different concentrations of compounds will be incubated with Menin^{M327I}/MOLM13 cells for 7 days, and viable cells will be detected using Promega's CellTiter-Glo assay.

MOLM13 cells were cultured in RPMI-1640 medium supplemented with 20% fetal bovine serum. One day prior to compound addition, cells were seeded into 96-well cell culture plates at 500 cells per well in a volume of 195 µL per well. Compounds were dissolved in DMSO to a final concentration of 10 mM. The 10 mM compound stock was then serially diluted 3-fold in DMSO to obtain 10 concentrations. For each concentration, 2 µL was taken and added to 48 µL of RPMI-1640 medium for a 25-fold dilution, and then added as a 40× compound to the prepared cell suspension. After co-incubation in the incubator for 7 days, 35 µL of Cell-Titer Glo was added, and the mixture was allowed to stand at room temperature for 10 minutes. Fluorescence signals were read on a CLARIO starPlus instrument, and finally the IC₅₀ value for cell proliferation inhibition was calculated using GraphPad Prism 5.0.

Note: * refers to multiplication, indicating a multiple.

**Table 2. Proliferation inhibitory activity of example compounds against MV-4-11 cells expressing wild-type Menin protein and MOLM13 cells expressing mutant Menin protein**

| **Example** | **IC₅₀ (nM)** | |
|---|---|---|
| | MV-4-11 | Menin^{M327I}/MOLM13 |
| **4** | 6.4 | 39.0 |
| **6** | 8.2 | 29.2 |
| **7** | 6.5 | 26.1 |
| **19** | 5.2 | 16.3 |
| **21** | 4.8 | 24.2 |
| **22** | 1.9 | 6.5 |
| **23** | 3.5 | 10.0 |
| **25** | 2.9 | 12.2 |
| **26** | 2.8 | 22.6 |
| **29** | 2.6 | 11.8 |
| **31** | 2.1 | 11.5 |
| **32** | 1.8 | 7.2 |
| **33** | 5.8 | 24.2 |
| **36** | 7.4 | 18.2 |
| **37** | 1.8 | 8.2 |
| **38** | 3.8 | 12.4 |

Table 2 lists the IC₅₀ values of the proliferation inhibitory activity of the example compounds of the present invention against MV-4-11 cells expressing wild-type Menin protein and MOLM13 cells expressing mutant Menin protein. The data indicate that the compounds provided by the present invention exhibit strong inhibitory activity against the proliferation of cells expressing both wild-type and mutant Menin protein.

### Pharmacokinetic study of compounds in SD rats

Male SD rats were obtained from Beijing Vital River Laboratory Animal Technology Co., Ltd. The rats were divided into groups of three animals each, and the test sample suspension (10 mg/kg, formulated in 0.5% MC + 0.1% SDS) was administered orally by gavage. The animals were fasted overnight prior to the experiment, with food withheld from 10 hours before dosing until 4 hours after dosing. Blood samples were collected at 0.25, 0.5, 1, 2, 4, 6, 8, and 24 hours after dosing. Following anesthesia with isoflurane using a small animal anesthesia machine, 0.3 mL of whole blood was collected from the retro-orbital venous plexus into heparin anticoagulant tubes. The samples were centrifuged at 4000 rpm for 5 minutes at 4°C, and the plasma was transferred to centrifuge tubes and stored at -80 °C until analysis. Plasma samples were extracted using the protein precipitation method, and the extracts were analyzed by LC/MS. The test results for some compounds are shown in Table 3.

**Table 3. Pharmacokinetic parameters of the example compounds.**

| **Parameter** | **Unit** | **PK Value (0.5% MC + 0.1% SDS, oral, 10 mg/kg)** | | | | | |
|---|---|---|---|---|---|---|---|
| | | **Ex. 4** | **Ex. 7** | **Ex. 21** | **Ex. 29** | **Ex. 31** | **Ex. 34** |
| T_{1/2} | hr | 9.16 | 15.2 | 21.2 | 14.3 | 11.7 | 9.09 |
| Tₘₐₓ | hr | 2.67 | 4.08 | 1.67 | 2.00 | 6.00 | 5.33 |
| Cₘₐₓ | ng/mL | 166 | 53.5 | 66.7 | 170 | 43.3 | 101 |
| AUC_{0-inf} | hr·ng/mL | 956 | 598 | 633 | 794 | 593 | 1491 |

Table 3 lists the pharmacokinetic data of the example compounds of the present invention in SD rats. It indicates that the compounds provided by the present invention exhibit relatively good in vivo metabolic profiles.

## Claims

1. A compound of formula (II) or a pharmaceutically acceptable salt, solvate, polymorph, tautomer, metabolite, or prodrug thereof, 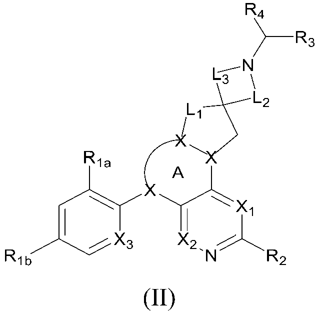 wherein,
Ring A is a 5- to 8-membered nitrogen-containing heterocyclic ring or nitrogen-containing heteroaromatic ring, each X is independently C or N,
R₁ₐ is H , -C(=O)-NR₂ₐR_{2b}, 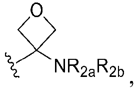 phenyl, or 5-6-membered heteroaryl, the heteroaryl contains one oxygen atom and/or one sulfur atom and/or 1-3 nitrogen atoms, and optionally contains carbonyl, and the phenyl and heteroaryl are optionally substituted by 3-6-membered cycloalkyl or C₁₋₄ alkyl,
R₂ₐ and R_{2b} are each independently H or C₁₋₄ alkyl, the alkyl is optionally substituted with -OH or -NH₂,
R_{1b} is F or Cl,
R₂ is selected from H, halogen, C₁₋₄ alkyl, -O-C₁₋₄ alkyl and -NR₃ₐR_{3b},
R₃ₐ and R_{3b} are each independently H or C₁₋₄ alkyl,
X₁ and X₂ are each independently N or CH,
X₃ is N or R₁₀,
R₁₀ is H, halogen, C₁₋₆ alkyl, or -O-C₁₋₆ alkyl,
L₁ is -CH₂-, -CH₂-CH₂- or -CH₂-CH₂-CH₂-,
L₂ and L₃ are each independently -CH₂- or -CH₂-CH₂-,
R₄ is C₁₋₆ alkyl, 3-8-membered cycloalkyl or 3-8-membered heterocycloalkyl,
R₃ is -C₁₋₆ alkylene -NR₄ₐR_{4b}, -C₁₋₆ alkylene -C(O)-NR₅ₐR_{5b}, -C₁₋₆ alkylene -OH, or -C₁₋₆ alkylene -NR₇-C(O)-O-C₁₋₄ alkylene -O-C(O)-C₁₋₄ alkyl, wherein the alkyl or alkylene are optionally substituted with halogen, -CN, -OH or -O-C₁₋₄ alkyl,
R₄ₐ and R_{4b} are each independently selected from H, C₁₋₆ alkyl, -C(O)-C₁₋₄ alkyl, -C(O)-O-C₁₋₄ alkyl and -C(O)-NR₈ₐR_{8b}, the C₁₋₆ alkyl is optionally substituted with halogen, -CN, -OH, -S(O)₂-C₁₋₄ alkyl, -O-C₁₋₄ alkyl, -C(O)-NR₆ₐR_{6b} or -NR_{6c}-C(O)-C₁₋₄ alkyl,
R₅ₐ, R_{5b}, R₆ₐ, R_{6b}, R_{6c}, R₇, R₈ₐ and R_{8b} are each independently selected from H and C₁₋₆ alkyl.

2. The compound according to claim 1 or a pharmaceutically acceptable salt, solvate, polymorph, tautomer, metabolite, or prodrug thereof, which has the structure represented by formula (I), 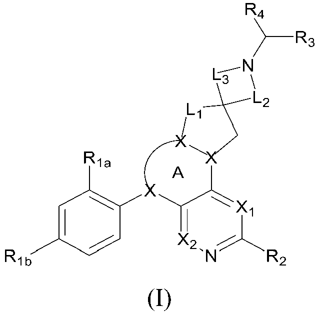 wherein,
Ring A is a 5- to 8-membered nitrogen-containing heterocyclic ring or nitrogen-containing heteroaromatic ring, each X is independently C or N,
R₁ₐ is -C(=O)-NR₂ₐR_{2b}, 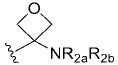 or 5-6-membered heteroaryl, wherein the heteroaryl contains **1-3** nitrogen atoms, and optionally contains a carbonyl group, and said heteroaryl is optionally substituted with 3-6-membered cycloalkyl or C₁₋₄ alkyl,
R₂ₐ and R_{2b} are each independently H or C₁₋₄ alkyl, the alkyl is optionally substituted with -OH or -NH₂,
R_{1b} is F or Cl,
R₂ is selected from H, halogen, C₁₋₄ alkyl, -O-C₁₋₄ alkyl and -NR₃ₐR_{3b},
R₃ₐ and R_{3b} are each independently H or C₁₋₄ alkyl,
X₁ and X₂ are each independently N or CH,
L₁ is -CH₂-, -CH₂-CH₂- or -CH₂-CH₂-CH₂-,
L₂ and L₃ are each independently -CH₂- or -CH₂-CH₂-,
R₄ is C₁₋₆ alkyl, 3-8-membered cycloalkyl or 3-8-membered heterocycloalkyl,
R₃ is -C₁₋₆ alkylene -NR₄ₐR_{4b}, -C₁₋₆ alkylene -C(O)-NR₅ₐR_{5b}, -C₁₋₆ alkylene -OH, or -C₁₋₆ alkylene -NR₇-C(O)-O-C₁₋₄ alkylene -O-C(O)-C₁₋₄ alkyl, wherein the alkyl or alkylene are optionally substituted with halogen, -CN, -OH or -O-C₁₋₄ alkyl,
R₄ₐ and R_{4b} are each independently selected from H, C₁₋₆ alkyl, -C(O)-C₁₋₄ alkyl, -C(O)-O-C₁₋₄ alkyl and -C(O)-NR₈ₐR_{8b}, the C₁₋₆ alkyl is optionally substituted with halogen, -CN, -OH, -S(O)₂-C₁₋₄ alkyl, -O-C₁₋₄ alkyl, -C(O)-NR₆ₐR_{6b} or -NR_{6c}-C(O)-C₁₋₄ alkyl,
R₅ₐ, R_{5b}, R₆ₐ, R_{6b}, R_{6c}, R₇, R₈ₐ and R_{8b} are each independently selected from H and C₁₋₆ alkyl.

3. The compound according to claim 2 or a pharmaceutically acceptable salt, solvate, polymorph, tautomer, metabolite, or prodrug thereof, wherein 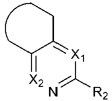 is

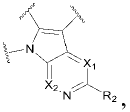

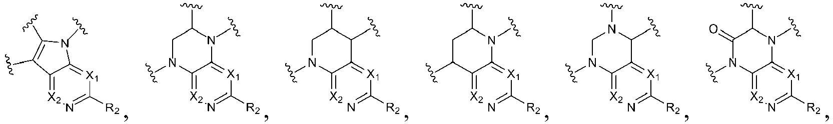

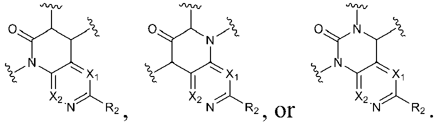

4. The compound according to claim 2 or a pharmaceutically acceptable salt, solvate, polymorph, tautomer, metabolite, or prodrug thereof, wherein 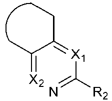 is 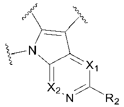 or

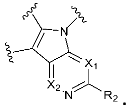

5. The compound according to claim 2 or a pharmaceutically acceptable salt, solvate, polymorph, tautomer, metabolite, or prodrug thereof, wherein R₁ₐ is -C(=O)-NR₂ₐR_{2b}, R₂ₐ and R_{2b} are each independently H or C₁₋₄ alkyl, R_{1b} is F, R₄ is isopropyl.

6. The compound according to claim 2 or a pharmaceutically acceptable salt, solvate, polymorph, tautomer, metabolite, or prodrug thereof, wherein R₂ is H or C₁₋₄ alkyl.

7. The compound according to claim 2 or a pharmaceutically acceptable salt, solvate, polymorph, tautomer, metabolite, or prodrug thereof, wherein R₃ is -C₁₋₆ alkylene -N(C₁₋₆ alkyl )-C₁₋₆ alkylene -O-C₁₋₄ alkyl.

8. The following compound or a pharmaceutically acceptable salt, solvate, polymorph, tautomer, metabolite, or prodrug thereof

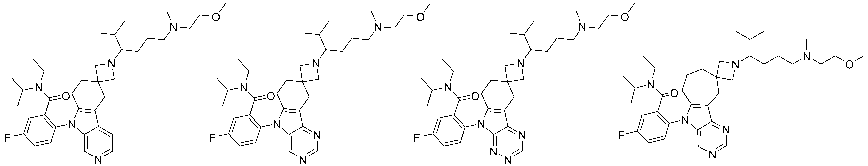

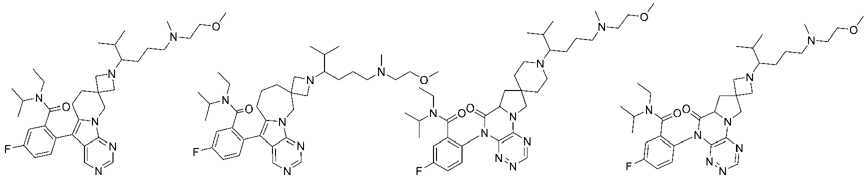

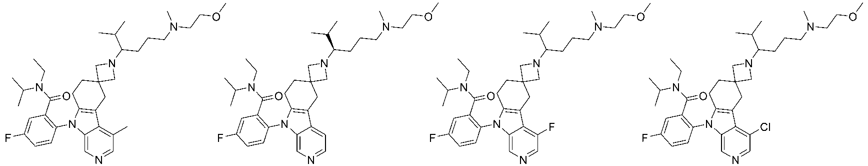

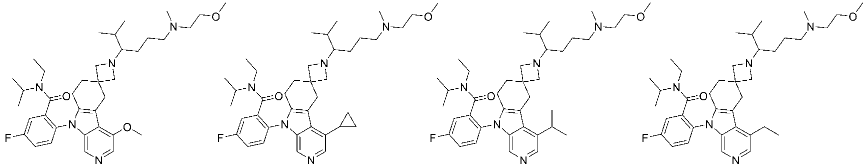

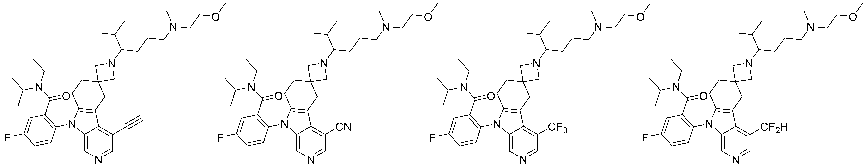

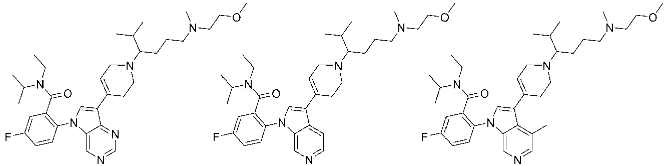

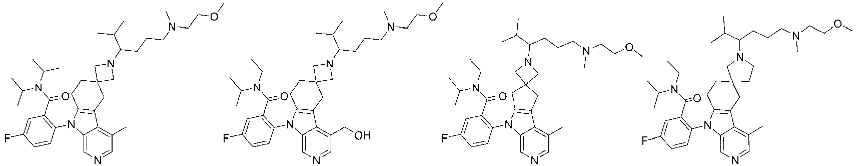

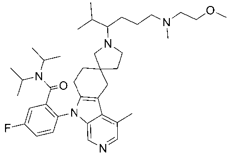

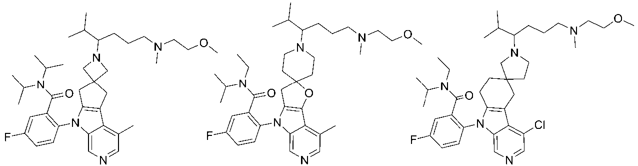

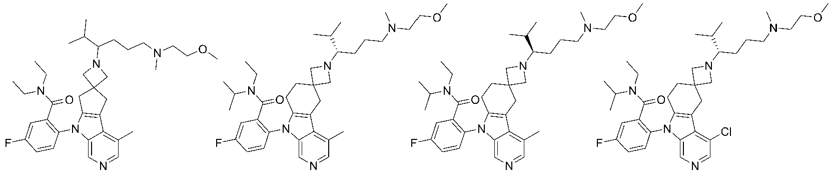

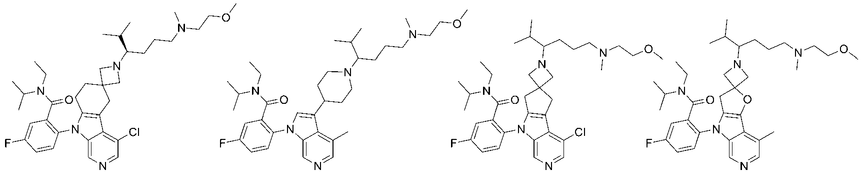

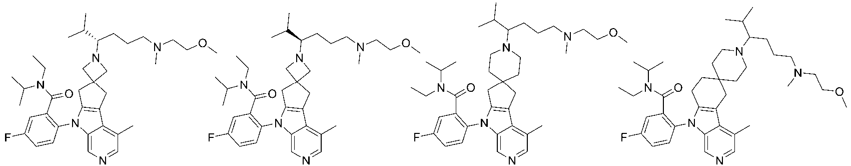

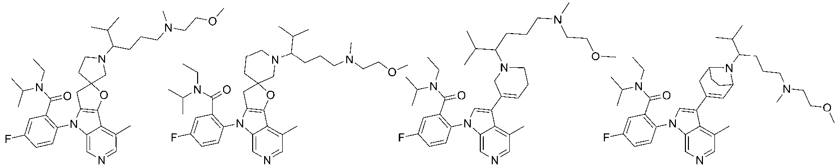

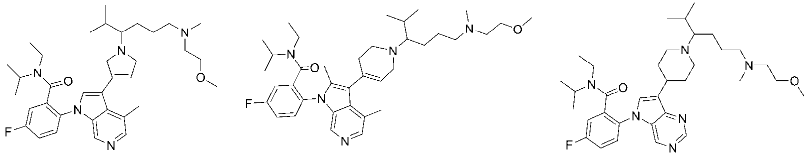

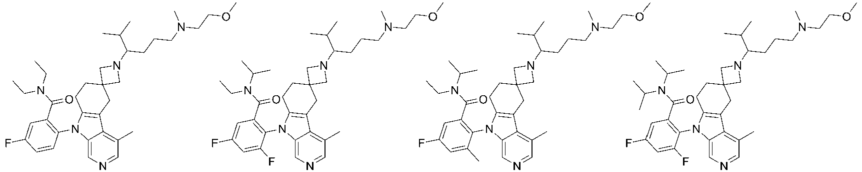

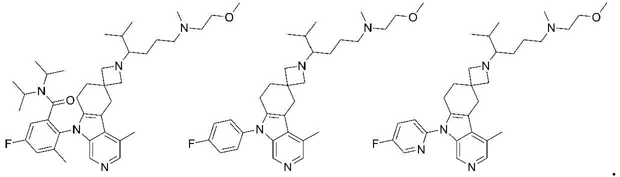

9. A pharmaceutical composition comprising the compound according to any one of claims 1 to 8 or a pharmaceutically acceptable salt, solvate, polymorph, tautomer, metabolite, or prodrug thereof, and optionally comprising a pharmaceutically acceptable carrier.

10. Use of the compound according to any one of claims 1 to 8 or a pharmaceutically acceptable salt, solvate, polymorph, tautomer, metabolite, or prodrug thereof, or the pharmaceutical composition according to claim 8 in the manufacture of a medicament for the treatment of a disease associated with MLL activity.

11. The use according to claim 10, wherein the disease associated with MLL activity is cancer, preferably acute leukemia (including MLL acute leukemia, MLL partial tandem duplication acute leukemia, NPM mutant acute leukemia, MOZ acute leukemia, NUP98 acute leukemia, and CALM acute leukemia), Chronic lymphocytic leukemia, chronic myeloid leukemia, myelodysplastic syndrome, polycythemia vera, malignant lymphoma (including B-cell lymphoma), myeloma (including multiple myeloma), Brain tumor, head and neck cancer, esophageal cancer, thyroid cancer, small cell lung cancer, non-small cell lung cancer, breast cancer, gastric cancer, gallbladder and bile duct cancer, liver cancer, hepatocellular carcinoma, Pancreatic cancer, colon cancer, rectal cancer, anal cancer, choriocarcinoma, endometrial cancer, cervical cancer, ovarian cancer, bladder cancer, urothelial carcinoma, Kidney cancer, renal cell carcinoma, prostate cancer, testicular tumor, testicular germ cell tumor, ovarian germ cell tumor, Wilms tumor, malignant melanoma, neuroblastoma, osteosarcoma, Ewing's sarcoma, chondrosarcoma, soft tissue sarcoma, or skin cancer.
